# EUROPEAN PATENT APPLICATION

(11) **EP 3 381 908 A1**
(43) Date of publication of application: **03.10.2018**
(21) Application number: 17163015.5
(22) Date of filing: 27.03.2017
(51) Int. Cl.: C07D 235/18, C07D 263/57, C07F 5/02, A61K 31/423, A61P 35/00

(54) **2-(4-(4-(BROMO-METHOXYBENZAMIDO)BENZYLAMINO)PHENYL)BENZAZOLE DERIVATIVES AND THEIR USE AS ANTI-HEPARANASE**

(71) Applicant: Leadiant Biosciences SA, 6850 Mendrisio (CH)
(72) Inventor: GIANNINI, Giuseppe, 00071 Pomezia (ROMA) (IT); BATTISTUZZI, Gianfranco, 00121 Ostia (ROMA) (IT); DI SANTO, Roberto, 00121 Ostia (ROMA) (IT); COSTI, Roberta, 00121 Ostia (ROMA) (IT); MADIA, Valentina Noemi, 88050 Simeri Crichi (CZ) (IT)
(74) Representative: Ghirardi, Valeria

(57) **Abstract**

The present invention relates to 2-(4-(4-(bromo-methoxybenzamido)benzylamino) phenyl)benzazole derivatives of formula 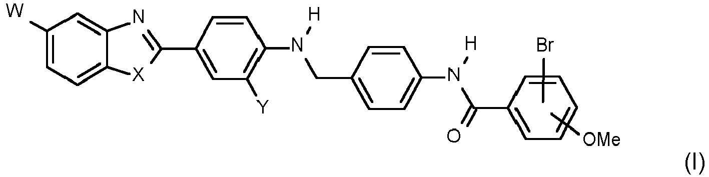 having an anti-heparanase activity.

The invention also relates to the use of such compounds as a medicament, in particular for the treatment of diseases and disorders associated with heparanase activity, and to pharmaceutical compositions comprising the same.

## Description

### FIELD OF THE INVENTION

The present invention relates to 2-(4-(4-(bromo-methoxybenzamido)benzylamino) phenyl)benzazole derivatives having an anti-heparanase activity.

The invention also relates to the use of such compounds as a medicament, in particular for the treatment of diseases and disorders associated with heparanase activity, and to pharmaceutical compositions comprising the same.

### BACKGROUND OF THE INVENTION

Heparan sulfate proteoglycans (HSPGs) are major components of the basement membrane and extracellular matrix. They are ubiquitous macromolecules associated with the cell surface and extracellular matrix (ECM) of a wide range of cells of vertebrate and invertebrate tissues (Bernfield M. et al. Annu. Rev. Biochem. 1999, 68, 729; lozzo R.V., San Antonio J.D. J. Clin. Invest. 2001, 108, 349; Kjellen L., Lindahl U. Annu. Rev. Biochem. 1991, 60, 443). HSPGs are a class of carbohydrate-modified proteins involved in key biological processes, including a role as co-receptors for a number of ligand molecules to regulate their signaling and distribution (Nakato H., Li J.P. Int. Rev. Cell. Mol. Biol. 2016, 325, 275).

Heparan sulfate (HS) is a component of cell surface and matrix-associated proteoglycans (HSPGs). A key function of HS is to bind and interact with signaling proteins, growth factors, plasma proteins, immune-modulators and other factors. In doing so, the HS chains and HSPGs are able to regulate protein distribution, bio-availability and action on target cells. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis (Billings P.C., Pacifici M. Connect Tissue Res. 2015, 56(4), 272). HS glycosaminoglycans bind to and assemble a multitude of ECM proteins (i.e., laminin, fibronectin, collagen type IV) and thereby contribute significantly to the ECM self assembly and integrity.

Heparanase is an endoglycosidase which cleaves heparan sulfate (HS) and hence participates in degradation and remodeling of the extracellular matrix (ECM), with a release of bioactive saccharide fragments and HS-bound growth factors and cytokines. Heparanase is preferentially expressed in human tumors and its over-expression in tumor cells confers an invasive phenotype in experimental animals. Heparanase upregulation correlates with increased tumor vascularity and poor postoperative survival of cancer patients. Heparanase is synthesized as a 65 kDa inactive precursor that undergoes proteolytic cleavage, yielding 8 kDa and 50 kDa protein subunits that heterodimerize to form an active enzyme.

Heparanase exhibits also non-enzymatic activities, independent of its involvement in ECM degradation.

There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R.et al. Matrix Biol. 2013, 32, 228; Ramani V.C. et al. FEBS J. 2013, 280, 2294; Vlodavsky I. et al. Matrix Biol. 2013. 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Rivara S., Milazzo F.M., Giannini G. *Future Med Chem.* 2016, *8(6),* 647. "Heparanase: a rainbow pharmacological target associated to multiple pathologies including rare diseases.").

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a viable therapeutic option.

Potent and selective heparanase inhibitors are therefore highly searched as therapeutic tools for those clinical indications in which heparanase inhibition is pharmacologically useful.

However, although in the last twenty years numerous efforts have been made in this sense and huge developments have been made in the knowledge of heparanase activity and functions, so far no drug able to inhibit or modulate heparanase functions has yet been registered (Rivara S. et al., Future Med Chem. 2016, 8(6), 647).

Some compounds are known in the art as heparanase inhibitors.

In particular, three classes of heparanase inhibitors are known: active analogous of endogenous substance, synthetic small molecule compounds and natural products. In the literature, monoclonal antibodies and nucleic acid-based inhibitors of heparanase are also mentioned.

Among these, only a few heparin derivatives have so far reached the stage of clinical trial (see for example the following reviews: Ferro V. et al. Mini Rev. Med. Chem. 2004, 4, 693; Jia L., Ma S. Eur. J. Med. Chem. 2016, 121, 209; Rivara S. et al. Future Med. Chem. 2016, 8, 647).

However, the heparin-derivatives macromolecules currently under clinical investigation have a high molecular weight.

Small molecules are instead particularly desirable due to their more favorable pharmacokinetic properties. Furthermore, they can be administered orally thus resulting in an improved patient compliance.

Indeed, progress in the development of drugs able to inhibit heparanase activity has been limited also by the lack of efficient small molecule inhibitors.

Therefore, there is still the need of small molecules able to inhibit heparanase activity.

In 2005, Courtney's group (Courtney S.M., Hay P.A., Buck R.T., Colville C.S., Phillips D.J., Scopes D.I., Pollard F.C., Page M.J., Bennett J.M., Hircock M.L., McKenzie E.A., Bhaman M., Felix R., Stubberfield C.R., Turner P.R. Bioorg. Med. Chem. Lett. 2005,15(9), 2295) synthetized and studied a series of benzoxazoles for their anti-heparanase activity.

Moreover, in Xu Y.J., Miao H.Q., Pan W., Navarro E.C., Tonra J.R., Mitelman S., Camara M.M., Deevi D.S., Kiselyov A.S., Kussie V, Wonga W.C. and Liua H., Bioorg. Med. Chem. Lett. 2006, 16, 404, N-(4-{[4-(1 H-Benzoimidazol-2-yl)-arylamino]-methyl}-phenyl)-benzamide derivatives are disclosed and their heparanase activity is investigated.

Small molecules inhibitors are also disclosed in the patent literature.

For example, WO2005042496 discloses (benzimidazol-2-yl)-phenyl-benzyl-amine derivatives for inhibiting heparanase activity.

WO2005030206 discloses aryl-1,3-azole derivatives as heparanase inhibitors.

WO2004046122 discloses benzoxazole and benzimidazole derivatives useful as inhibitors of heparanase.

All the above mentioned documents show that a big effort has been put in the design of small molecules compounds able to efficiently inhibit heparanase and also endowed with good pharmacokinetic properties. It is therefore evident that such molecules are still highly desired.

### SUMMARY OF THE INVENTION

It has now been found that 2-(4-(4-(bromo-methoxybenzamido)benzylamino)phenyl)-benzazole derivatives represent a new class of compounds with ability to inhibit efficiently heparanase activity.

In particular, it has been found that the presence of fluorine (F) on the phenyl ring linked to the benzazole moiety, as shown in below formula (I), provides the compound with an increased heparanase inhibition activity with respect to similar unsubstituted compounds.

It is therefore an object of the present invention a compound having the following formula (I) 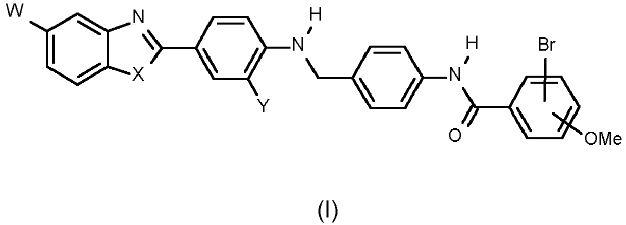 wherein
Y is fluorine,
X is selected from the group consisting of O and NH,
W is selected from the group consisting of H and CH₂COZ, wherein Z is selected from the group consisting of OH, NH-CHR-COOH, wherein R is the residue of an amino acid, and NHCH₂B(OH)₂,
the enantiomers, diastereoisomers, and mixtures thereof,
or a pharmaceutically acceptable salt, hydrate or solvate thereof.

Indeed, it has been found that such compounds are able to inhibit heparanase activity.

In a preferred embodiment of the invention, X is NH and W is H.

In another preferred embodiment, X is NH and W is CH₂COZ wherein Z is NH-CHR-COOH and R is an amino acid residue, preferably of glycine.

In another preferred embodiment, X is O and W is CH₂COZ wherein Z is NH-CHR-COOH and R is an amino acid residue, preferably of glutamic acid.

When Z is NH-CHR-COOH, R is preferably a residue of an amino acid selected from glycine, alanine and phenylalanine.

A process for the preparation of the compound of formula (I), as will be better defined below, is also an object of the present invention.

It is also an object of the invention the compound of formula (I) for use as a medicament.

A further object of the present invention is a compound of formula (I) for use for the treatment of a disease which can be improved or prevented by an anti-heparanase activity.

In a preferred embodiment, said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, glucose induced following peritoneal dialysis) and aging.

A further object of the invention is a pharmaceutical composition containing as active ingredient a compound of formula (I) and at least one pharmaceutically acceptable vehicle and/or excipient.

### Detailed description of the invention

### Definitions

Within the meaning of the present invention, the term "anti-heparanase activity" refers to an activity sufficient to inhibit partially or totally the enzyme heparanase.

Within the meaning of the present invention, for "residue of an amino acid" it is intended the characterizing portion of an amino acid. Said portion is specific to each amino acid. The structure of the characterizing group for each known amino acid is commonly known in the art.

It has been found that compounds of formula (I) have an effect of inhibition on heparanase activity. In particular, it has been found that the presence of fluorine (F) in position 3 of the phenyl ring linked to the azole moiety, as shown in formula (I) above, provides for an endowed heparanase activity.

This is evident, for example, from the following table 1 showing that the introduction of a fluorine instead of an hydrogen in position 3 of phenyl ring linked to azole moiety considerably increases the heparanase inhibitory activity of the fluorine compound with respect to the corresponding unsubstituted compound (Y=H). Heparanase activity is measured by AGAIA assay (Hammond et al. Anal. Biochem. 2010, 396, 112) and suramine is taken as reference compound due to its known heparanase inhibition activity. SST0408AA1 is a known compound (Biorg. & Med. Chem. Lett. 2006, 16, 404, ImClone) and new compound SST0623AA1 is its fluorine derivative. Compounds SST0914AA1 and SST0915AA1 are not within the scope of the present invention but they are used as reference compounds to show the positive effect of the introduction of fluorine on heparanase inhibition activity.

**Table 1. IC₅₀ value in AGA*IA assay, indicative of heparanase inhibition. Comparison of fluorinated (compound of the present invention) and non-fluorinated analogues.**

| Structural formula | Internal code | Y | AGA*IA assay IC₅₀ (nM) | Notes |
|---|---|---|---|---|
| *Reference compound* | Suramin | | ∼ 26,000 | Prior art compound |
| 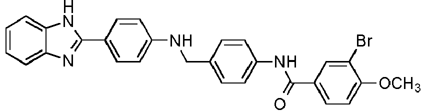 | SST0408AA 1 | H | 600 | Prior art compound |
| 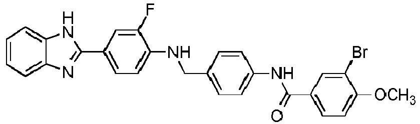 | **SST0623AA1** | **F** | **165** | **Fluorine derivative of SST0408AA1** |
| 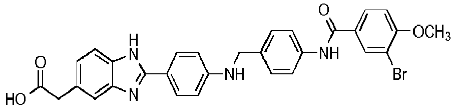 | SST0914AA1 | H | >10000 | Compound not part of the invention |
| 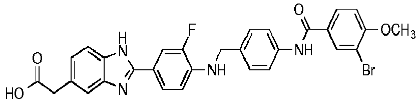 | **SST0867AA1** | **F** | **2860** | **Fluorine derivative of SST0914AA1** |
| 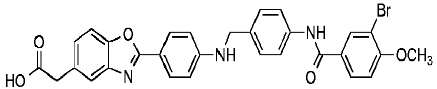 | SST0915AA1 | H | >10000 | Compound not part of the invention |
| 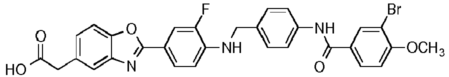 | **SST0864AA1** | **F** | **2560** | **Fluorine derivative of SST0915AA1** |

In the compounds of the invention, Y is always fluorine.

In the compounds of formula (I) W can be selected from H, CH₂COOH, CH₂CONH-CHR-COOH, wherein R is the residue of an amino acid, and CH₂CONHCH₂B(OH)₂.

In an embodiment of the invention, X is NH and W can have any of the meanings above defined. In particular, in this embodiment W is preferably H or CH₂COZ wherein Z is NH-CHR-COOH and R is an amino acid residue. More preferably, said amino acid residue is from glycine.

In an embodiment of the invention, X is O and W can have any of the meanings above defined. Preferably, in this embodiment W is CH₂COOH or CH₂COZ wherein Z is NH-CHR-COOH and R is an amino acid residue. More preferably, said amino acid residue is from glutamic acid.

When Z is NH-CHR-COOH, R can be the residue of any amino acid. In particular, it can be from a D-amino acid or a L-amino acid. Also, it can be from a natural or unnatural (e.g. synthetic) amino acid. The structure of the R group for each known amino acid is well known in the field. For example, the R group of glycine is H, the R group of alanine is CH₃, the R group of valine is CH(CH₃)₂, and so on.

In particular, R can be the residue of any amino acid selected from the group consisting of: alanine (Ala), arginine (Arg), asparagine (Asn), aspartic acid (Asp), cysteine (Cys), glutamic acid (Glu), glutamine (Gln), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val).

Preferably, it is from an amino acid selected from the group consisting of: glycine (Gly), alanine (Ala), phenylalanine (Phe) and glutamic acid (Glu).

OMe in formula (I) represents a O-methyl group.

Br in formula (I) represents a bromine.

In the compound of formula (I) the groups bromine (Br) and O-methyl (OMe) can be present on any free position of the phenyl ring.

In a preferred embodiment, the bromine is in position 3 and the OCH₃ group is in position 4 of the phenyl ring.

Any nitrogen group can be protected. The protective group is preferably selected from tert-butoxycarbonyl, benzyloxycarbonyl and 9-fluorenylmethyloxycarbonyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I) and inorganic bases, such as sodium, potassium, calcium and aluminum hydroxides, as well as those with organic bases such as, for example, lysine, arginine, N-methyl-glucamine, triethylamine, triethanolamine, dibenzilamine, methylbenzilamine, di-(2-ethyl-hexyl)-amine, piperidine, N-ethylpiperidine, N,N-di-ethylaminoethylamine, N-ethylmorpholine, 3-phenethylamine, N-benzil-3-phenethylamine, N-benzil-N,N-dimethylamine. A preferred salt is sodium salt.

Also, pharmaceutically acceptable hydrates or solvates of the compound of formula (I) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Preferred compounds according to the present invention are the followings:
2-(4-(4-(3-bromo-4-methoxybenzamido)benzyl)amino)-3-fluorophenyl)-1*H*-benzo[*d*]imidazole (22) - (SST0623AA1)
2-(2-(4-((4-(3-bromo-4-methoxybenzamido)benzyl)amino)-3-fluorophenyl)benzo[d]oxazol-5-yl)acetic acid (30) - (SST0864AA1)
(2-(2-(4-((4-(3-bromo-4-methoxybenzamido)benzyl)amino)-3-fluorophenyl)benzo[d]oxazol-5-yl)acetyl)glycine (37) - (SST0865AA1)
2-(2-(4-((4-(3-bromo-4-methoxybenzamido)benzyl)amino)-3-fluorophenyl)-1*H-*benzo[d]imidazol-5-yl)acetic acid (10) - (SST0867AA1)
((2S)-2-(2-(4-((4-(3-bromo-4-methoxybenzamido)benzyl)amino)-3-fluorophenyl)-1*H-*benzo[*d*]imidazol-5-yl)acetyl)-alanine (16) - (SST0868AA1)
((2S)-2-(2-(4-((4-(3-bromo-4-methoxybenzamido)benzyl)amino)-3-fluorophenyl)-1*H-*benzo[*d*]imidazol-5-yl)acetyl)-phenylalanine (17) - (SST0869AA1)
((2-(2-(4-((4-(3-Bromo-4-methoxybenzamido)benzyl)amino)-3-fluorophenyl)-1 H-benzo[d]imidazol-5-yl)acetamido)methyl)boronic acid (19) - (SST0873AA1)
2-(2-(2-(4-((4-(3-bromo-4-methoxybenzamido)benzyl)amino)-3-fluorophenyl)-1 H-benzo[*d*]imidazol-5-yl)acetamido)acetic acid (15) - (SST0871AA1) and
((2S)-2-(2-(4-((4-(3-bromo-4-methoxybenzamido)benzyl)amino)-3-fluorophenyl)benzo[d]oxazol-5-yl)acetyl)-glutamic acid (40) - (SST0876AA1).

Further compounds according to the present invention are:
((2S)-2-(2-(4-((4-(3-bromo-4-methoxybenzamido)benzyl)amino)-3-fluorophenyl)benzo[d]oxazol-5-yl)acetyl)-alanine (38) - (SST0874AA1),
((2S)-2-(2-(4-((4-(3-bromo-4-methoxybenzamido)benzyl)amino)-3-fluorophenyl)benzo[d]oxazol-5-yl)acetyl)-phenylalanine (39) - (SST0875AA1),
((2S)-2-(2-(4-((4-(3-bromo-4-methoxybenzamido)benzyl)amino)-3-fluorophenyl)benzo[d]oxazol-5-yl)acetyl)-lysine (41) - (SST0916AA1), and
((2-(2-(4-((4-(3-Bromo-4-methoxybenzamido)benzyl)amino)-3-fluorophenyl)benzo[d]oxazol-5-yl)acetamido)methyl)boronic acid (43) - (SST0877AA1).

The compounds of the present invention can be synthesized by many methods available to those skilled in the art of organic chemistry. General and exemplary synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter.

The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecules should be consistent with the transformations proposed.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Also, the skilled in the art can easily alter the reagents and reaction conditions exemplified in the schemes below to include any combination of substituents as defined above. Also, the skilled artisan can easily use interchangeable steps for each synthetic process and incorporate isolation and/or purification steps as deemed necessary.

Starting materials useful for the preparing the compounds of the invention and intermediates therefor, are commercially available or can be prepared by well known synthetic methods.

The final products obtained by the synthesis described below may be purified using techniques commonly known to one skilled in the art such as preparatory chromatography, thin-layer chromatography, HPLC, or crystallization.

Exemplary processes for the synthesis of the compounds of the invention are herein described.

### Exemplary processes

### Benzimidazoles

Exemplary processes to obtain compounds according to the present invention wherein X is NH are herein disclosed.

In particular, an exemplary process for obtainment of compounds of formula (I) wherein X is NH and W is CH₂COZ and Z is OH is herein disclosed and represented in the following scheme 1.

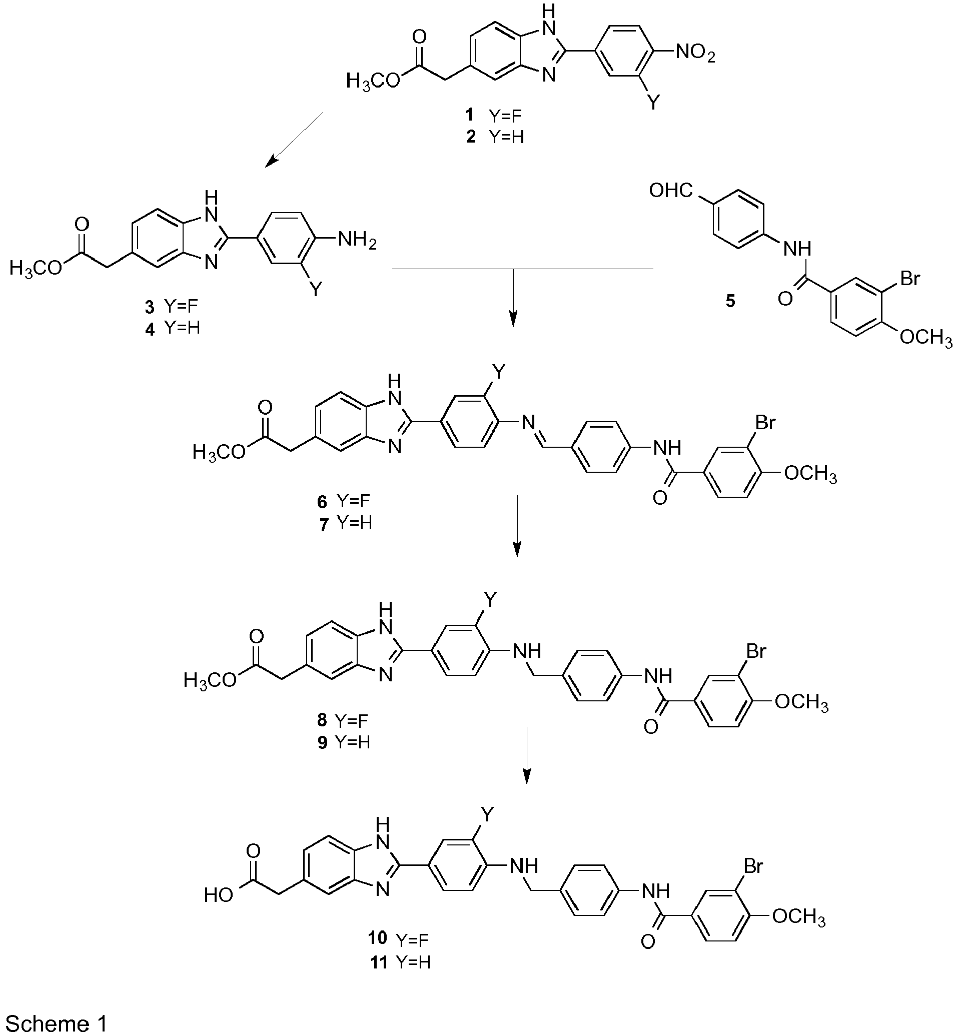

Compound 3 is obtained by reduction of methyl 2-(2-(3-fluoro-4-nitrophenyl)-1*H-*benzo[*d*]imidazol-5-yl)acetate (1) (obtained according to Bahrami, K. et al, J. Org. Chem. 2008, 73(17), 6835) using common reduction means, for example diethylamine and ammonium formate, in a suitable solvent, for example ethyl acetate or methanol. Palladium on carbon (Pd/C) can be used as a catalyst. Compound 6 is then obtained by condensation of compound 3 with 3-bromo-N-(4-formylphenyl)-4-methoxybenzamide (5) (obtained according to Liu, H., Pan, W., Xu, Y.J. WO 2005042496 A1 20050512 and Xu, Y.J. et al. Bioorg. & Med. Chem. Lett. 2006. 16(2), 404), for example by refluxing them in toluene in the presence of p-toluenesulfonic acid. The imino function of compound 6 is reduced with suitable reduction means, for example sodium borohydride, and compound 8 is obtained.

The terminal alkyl ester of compound 8 is hydrolysed by suitable means, for example using lithium hydroxide, and compound 10 (Z = OH) is obtained.

Compounds wherein Y = H have also been synthetized with the same process using suitable starting compounds; such compounds are not within the scope of the present invention.

Compounds of formula (I) wherein X is NH and W is CH₂COZ wherein Z comprises an amino acid residue can be obtained starting from the above mentioned compound 10 and adding the desired amino acid, usually in esterified form, for example as commercially available amino ester hydrochloride. The reaction can be carried out in presence of suitable reagents, for example catalysts or activators, such as 1-ethyl-3-(3-dimethylaminopropyl)carbodiimide (EDCI), 4-dimethylaminopyridine (DMAP) and N,N-Diisopropylethylamine (DIPEA), in a suitable solvent, such as an anhydrous mixture of tetrahydrofuran/N,N-dimethylformamide. Finally, the ester present on the amino acid residue is hydrolysed using a suitable reagent, for example LiOH, to obtain the desired amino acid derivative (compounds 15-17), as represented in the following scheme 2.

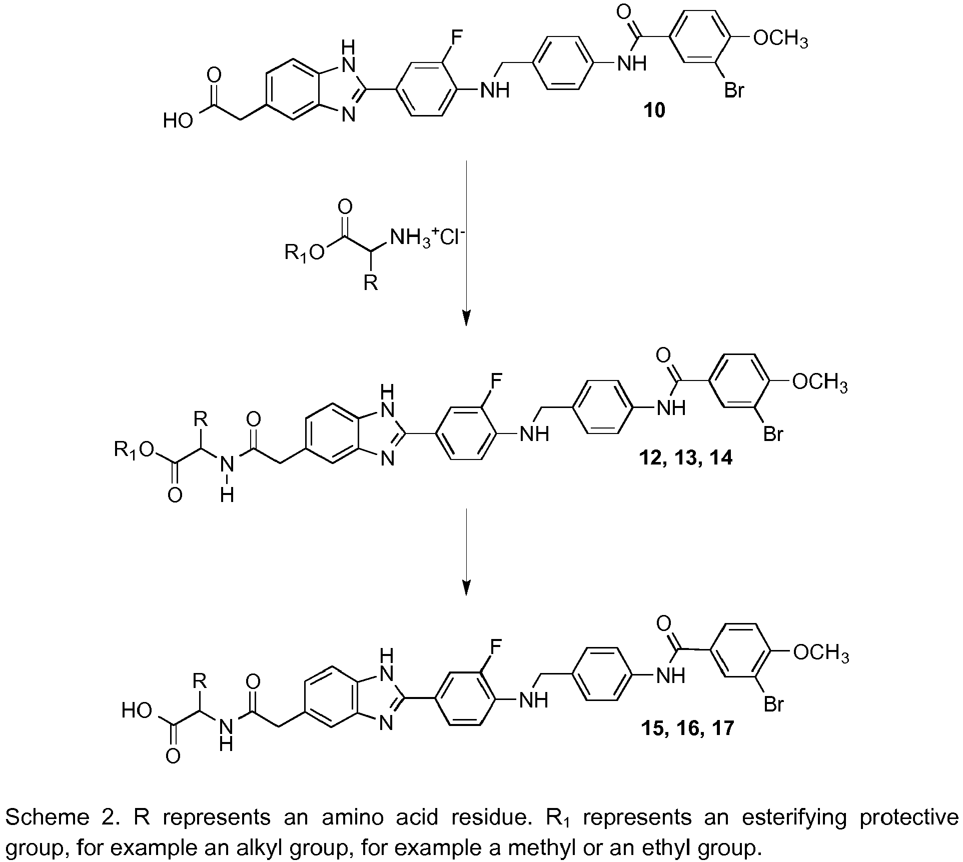

An exemplary process to obtain compounds of formula (I) wherein X is NH and W is CH₂COZ, wherein Z is NHCH₂B(OH)₂ is herein described and represented in the following scheme 3.

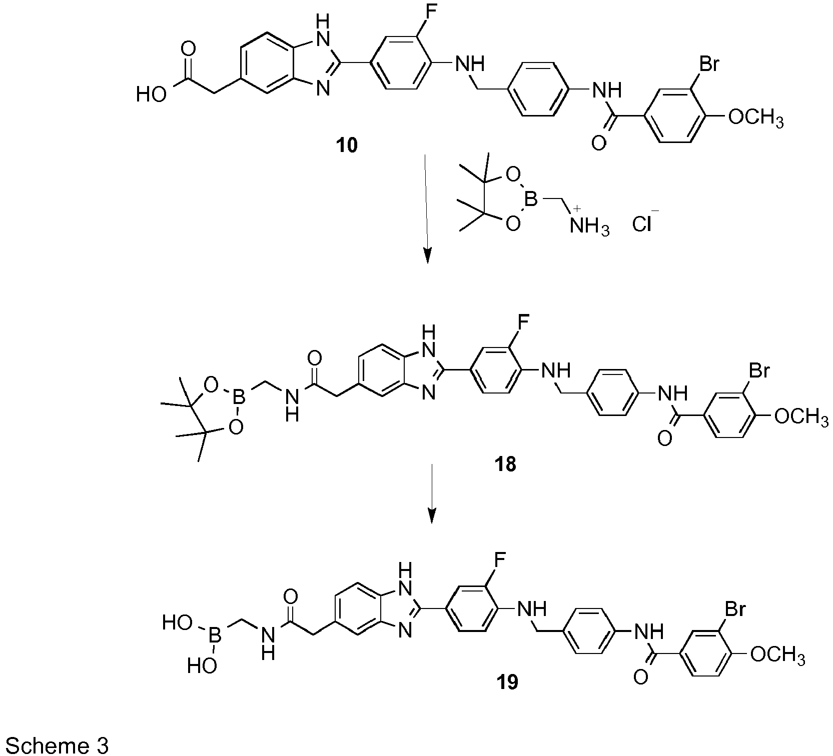

Compound 18 can be obtained starting from the above mentioned compound 10 and adding commercially available (4,4,5,5-tetramethyl-1,3,2-dioxaborolan-2-yl)methanaminium chloride and suitable reagents, such as for example O-(benzotriazol-1-yl)-N,N,N',N'-tetramethyluronium tetrafluoroborate (TBTU). The reaction can be carried out in suitable solvents, such as anhydrous tetrahydrofuran, and the mixture is cooled to a temperature comprised between -60°C and -100°C. A suitable reagent such as N,N-diisopropylethylamine (DIPEA), in suitable solvents, for example anhydrous tetrahydrofuran, is then added. The ester function on the boronate residue is then removed using suitable reagents, for example ammonium acetate and sodium periodate in a suitable solvent such as tetrahydrofuran, to obtain compound 19.

An exemplary process for obtainment of compounds of formula (I) wherein X is NH and W is H is herein described and represented in the following scheme 4.

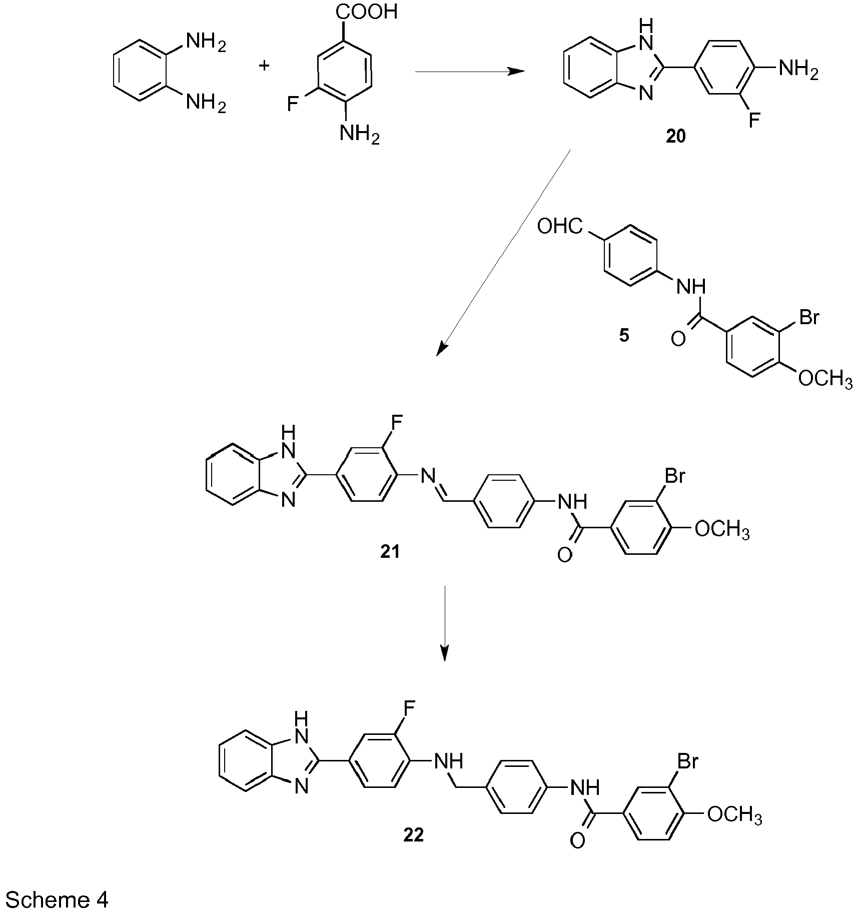

A condensing agent, for example polyphosphoric acid, is added to commercially available o-phenylendiamine and 4-amino-3-fluorobenzoic acid to obtain compound 20. Reaction is usually carried out at about 220°C. The terminal amine is then condensed with the aldehyde function of compound 5 (obtained according to Liu, H., Pan, W., Xu, Y.J. WO 2005042496 A1 20050512 and Xu, Y.J. et al. Bioorg. & Med. Chem. Lett. 2006. 16(2), 404), for example using acetic acid as a catalyst, to obtain compound 21. The imino function is then reduced using a suitable reagent, for example sodium borohydride, to obtain the desired final derivative (compound 22).

### Benzoxazoles

Exemplary processes to obtain compounds according to the present invention wherein X is O are herein disclosed.

In particular, an exemplary process for obtainment of compounds of formula (I) wherein X is O and W is CH₂COZ wherein Z have any of the meanings above defined is herein described.

An exemplary procedure for obtaining compounds wherein Z is OH or comprises an amino acid residue is represented in the following scheme 5.

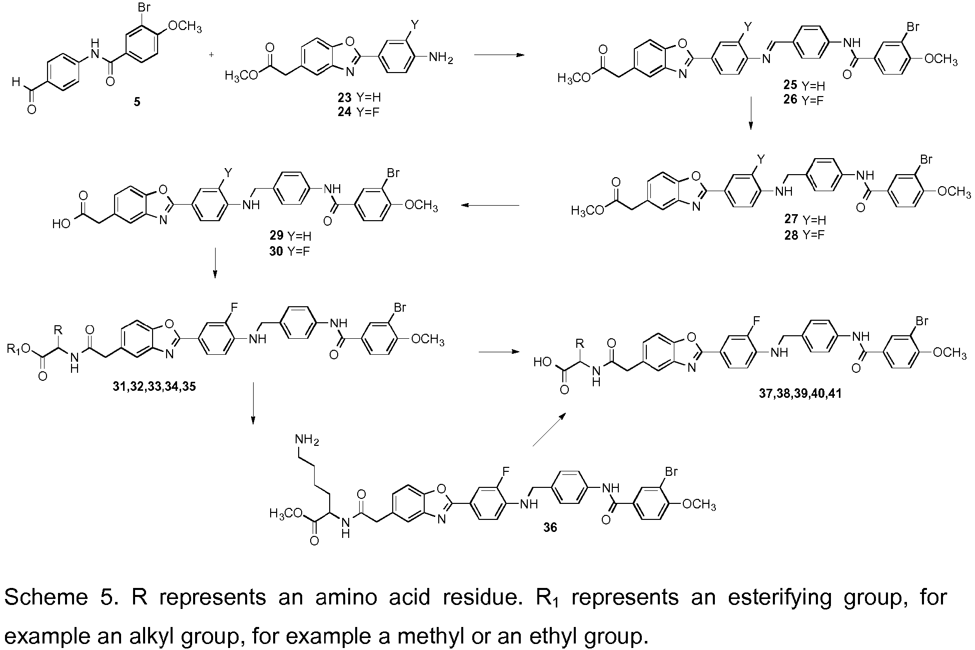

Compound 26 is obtained by condensation of amine 24 (obtained according to WO2004046122) and aldehyde 5 (obtained according to Liu, H., Pan, W., Xu, Y.J., WO2005042496A1 and Xu, Y.J. et al. Bioorg. & Med. Chem. Lett. 2006. 16(2), 404), for example refluxing in the appropriate solvent, for example toluene, with an appropriate catalyst, for example p-toluenesulfonic acid. Reaction can be carried out at a temperature of about 150°C. The imino function is then reduced using a suitable reagent, for example sodium borohydride, to obtain the desired compound 28. The terminal alkyl ester of compound 28 is hydrolysed, for example with lithium hydroxide, and compound 30 (Z = OH) is obtained.

To obtain compounds wherein Z comprises an amino acid residue, the desired amino acid is added, usually in esterified form, for example as commercially available amino ester hydrochloride, and compounds 31-35 are obtained. The reaction can be carried out in presence of suitable reagents, for example catalysts or activators, such as 1-ethyl-3-(3-dimethylaminopropyl)carbodiimide (EDCI), 4-dimethylaminopyridine (DMAP) and N,N-Diisopropylethylamine (DIPEA), in a suitable solvent, such as anhydrous tetrahydrofuran. If the residue R comprises an amino group this is usually in a protected from and subsequent deprotection of said amino group is carried out with usual means, for example using piperidine in anhydrous N,N-dimethylformamide; in the scheme 5 above it is exemplified a case in which the amino acid residue R is from a lysine and an intermediate step of deprotection lead to compound 36, wherein the NH group is deprotected. Finally, the ester present on the acidic function of the amino acid residue is hydrolysed using a suitable reagent, for example LiOH, to obtain the desired amino acid derivatives (compounds 37-41).

An exemplary process to obtain compounds wherein X is O and W is CH₂COZ, wherein Z is NHCH₂B(OH)₂ is represented in the following scheme 6.

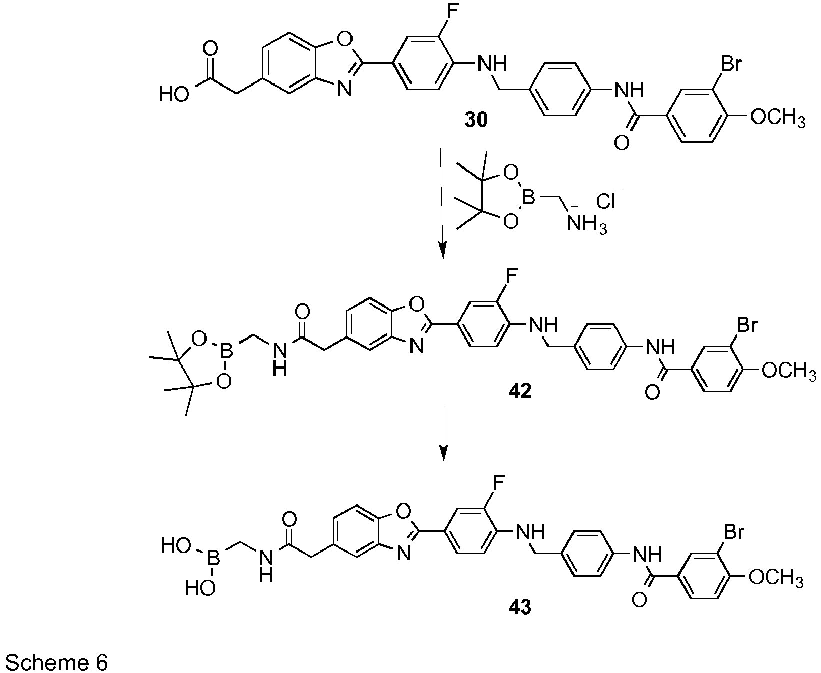

Compound 42 can be obtained starting from the above mentioned compound 30 and adding commercially available (4,4,5,5-tetramethyl-1,3,2-dioxaborolan-2-yl)methanaminium chloride, and suitable reagents, such as for example O-(benzotriazol-1-yl)-N,N,N',N'-tetramethyluronium tetrafluoroborate (TBTU), and N,N-diisopropylethylamine (DIPEA) in suitable solvents, for example N,N'-dimethylformamide at a temperature comprised between around -60 and around -100°C. The ester function on the boronate residue can be removed using a suitable reagent, for example ammonium acetate and sodium periodate, to obtain compound 43.

An exemplary process to obtain compounds wherein X is O and W is H, is represented in the following scheme 7.

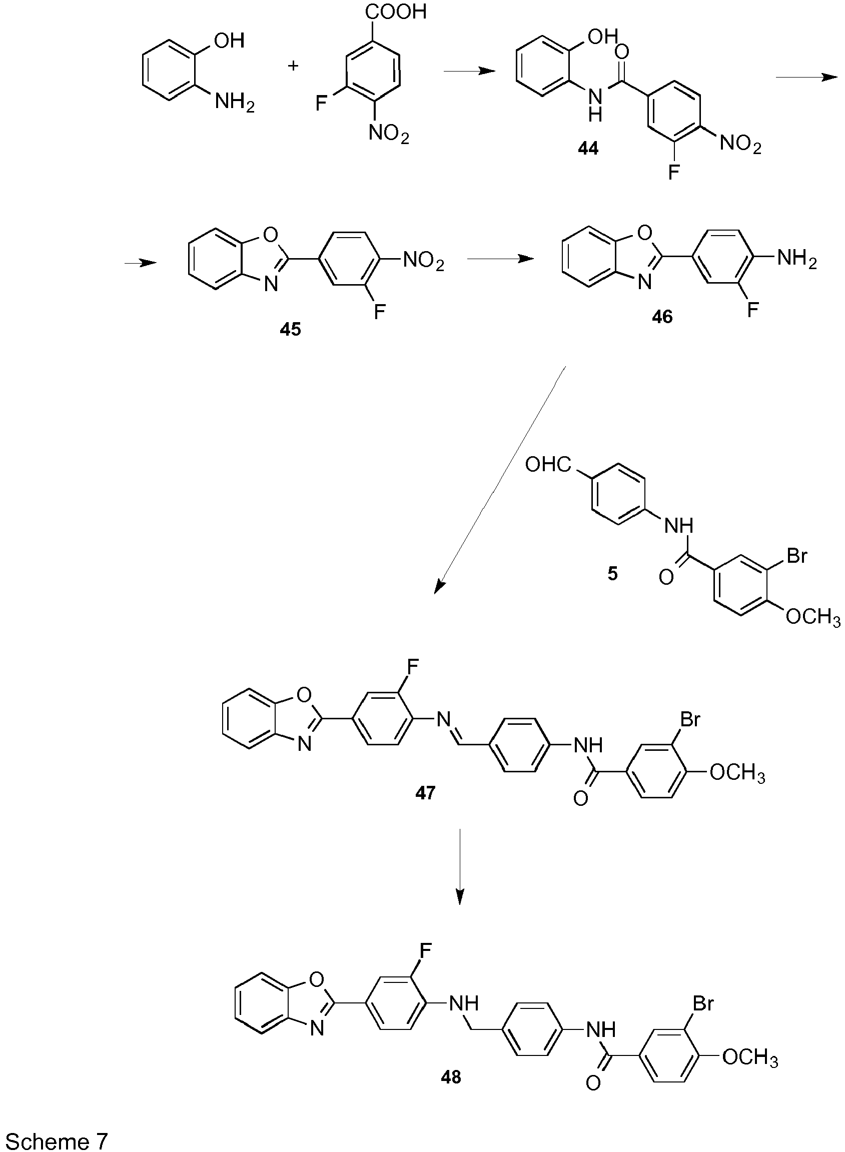

Compound 45 is obtained as previously mentioned according to WO2004046122 starting from o-aminophenol and 3-fluoro-4-nitrobenzoic acid. Compound 46 is obtained by reduction of compound 45 using common reduction means, for example diethylamine and ammonium formate.

Condensation of compound 46 with 3-bromo-N-(4-formylphenyl)-4-methoxybenzamide 5 (obtained according to Liu, H., Pan, W., Xu, Y.J. WO 2005042496 A1 20050512 and Xu, Y.J. et al. Bioorg. & Med. Chem. Lett. 2006. 16(2), 404), is obtained for example by refluxing them in toluene in the presence of p-toluenesulfonic acid. The imino function of compound 47 is reduced with suitable reduction means, for example with sodium borohydride, and compound 48 is obtained.

All the transformations above described are only exemplary synthetic procedures already known in organic chemistry and well known to the experts in the field.

Any variation or combination of substituents of compounds of formula (I) which is within the scope of the present invention and is not explicitly shown or described in the above processes, can be easily obtained starting from the exemplary processes described above with suitable modifications, for example in the starting compounds or reagents, that are well-known within the knowledge of the skilled person in the field of organic chemistry. Reference can be made, for example, to the handbook "March's Advanced Organic Chemistry, Reactions, Mechanism and structures.", Michael B. Smith, Jerry March, last edition.

For example, in all the above schemes the bromine and methoxy groups are represented in positions 3 and 4, respectively, of the phenyl ring. Compounds having bromine and methoxy groups on other positions of the phenyl ring are still within the scope of the invention and can be obtained by using in the above processes the suitable analogue of 3-bromo-N-(4-formylphenyl)-4-methoxybenzamide (5).

### Medical uses

According to the present invention, the compounds of formula (I) can be used as medicaments.

In particular, according to the present invention, the compounds of formula (I) are endowed with anti-heparanase activity. Therefore, they can be advantageously used for the treatment of diseases which can be improved or prevented by the inhibition of heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist. Updat. 2016, 29, 54). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (I) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Specific heparanase inhibition reverses glucose-induced mesothelial-to-mesenchymal transition Masola .V et al *Nephrol. Dial. Transplant* 2017; 1-9) and thus heparanase inhibition may be also beneficial in those pathologic processes involving EMT, as fibrosis occurring at different organs.

Examples of diseases which can be treated by compounds of formula (I) are tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

The skilled in the art, for example a physician, can identify and recognize if a disease can be ameliorated by an anti-heparanase activity on the basis of the common knowledge in the field. For example, for a broad, even though not exhaustive, overview, on possible clinical applications of heparanase inhibitors, reference can be made to the previously mentioned reviews of Rivara et al., 2016 and Vlodavski et al., 2016.

Also, tests are known in the field and available to the skilled person to evaluate if a compound is endowed with an anti-heparanase activity, for example the AGAIA assay (Hammond E. et al. Anal. Biochem. 2010, 396, 112).

Compounds of the invention are particularly advantageous also for non-tumor applications since they are endowed with a strong anti-heparanase activity and therefore they can already be efficiently used at very low doses at which they are not cytotoxic. Indeed, they are selective heparanase inhibitors already at concentrations in which they do not interfere significantly with cell proliferation. This renders them particularly useful for therapeutic applications wherein a cytotoxic effect is undesired. Also, it has been found that such compounds at not cytotoxic concentrations are already able to inhibit invasion of tumor cells thus being particularly useful in the treatment of tumors and metastasis, also as adjuvants of chemotherapeutic agents. Furthermore, the compounds of the invention have also an inhibitory effect against tumor transcription genes encoding for proangiogenic factors thus further supporting their therapeutic use in cancer diseases.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (I) as an active ingredient, in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The composition according to the present invention contains, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical composition according to the invention comprising the compound of formula (I) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of the invention act by modifying the tumor microenvironment to affect cell growth and spread as well as to facilitate the tumor killing action of other agents. Therefore, the combination of the compounds of the invention with any chemotherapeutic agent can be particularly useful. The chemotherapeutic agent can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide.

For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

According to the administration route chosen, the compositions will be in solid or liquid form, suitable for oral, parenteral or topical administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (I) with suitable excipients, stabilizers and/or pharmaceutically acceptable diluents.

It is also an object of the invention the use of such pharmaceutical composition as a medicament, in particular in the treatment of a disease which can be improved or prevented by an anti-heparanase activity. More in particular, the above mentioned diseases can be treated by such pharmaceutical compositions.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S., Ono T., Aoki H., Amano S. Dermatol Sci. 2011, 64(3), 223).

In view of the above and of the heparanase inhibition activity of the compounds of the invention, it is also an object of the present invention the cosmetic use of the compounds of formula (I) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity. In particular, due to their potent anti-heparanase activity, they can already exert their effect at very low doses, at which they are not cytotoxic and thus suitable for cosmetic uses.

It is also an object of the invention, a cosmetic composition comprising the compound of formula (I) and cosmetically acceptable excipients and/or vehicles. Such excipients or vehicles can be chosen by the skilled person among the cosmetic ingredients commonly used in the field. In particular, they are components commonly used in external preparations. Such cosmetic composition can be used in the prevention or suppression of wrinkles or in any other anti-aging applications. It is preferably applied and administered topically.

The following examples further illustrate the invention.

### EXAMPLES

### Example 1

### Compounds 10, 11

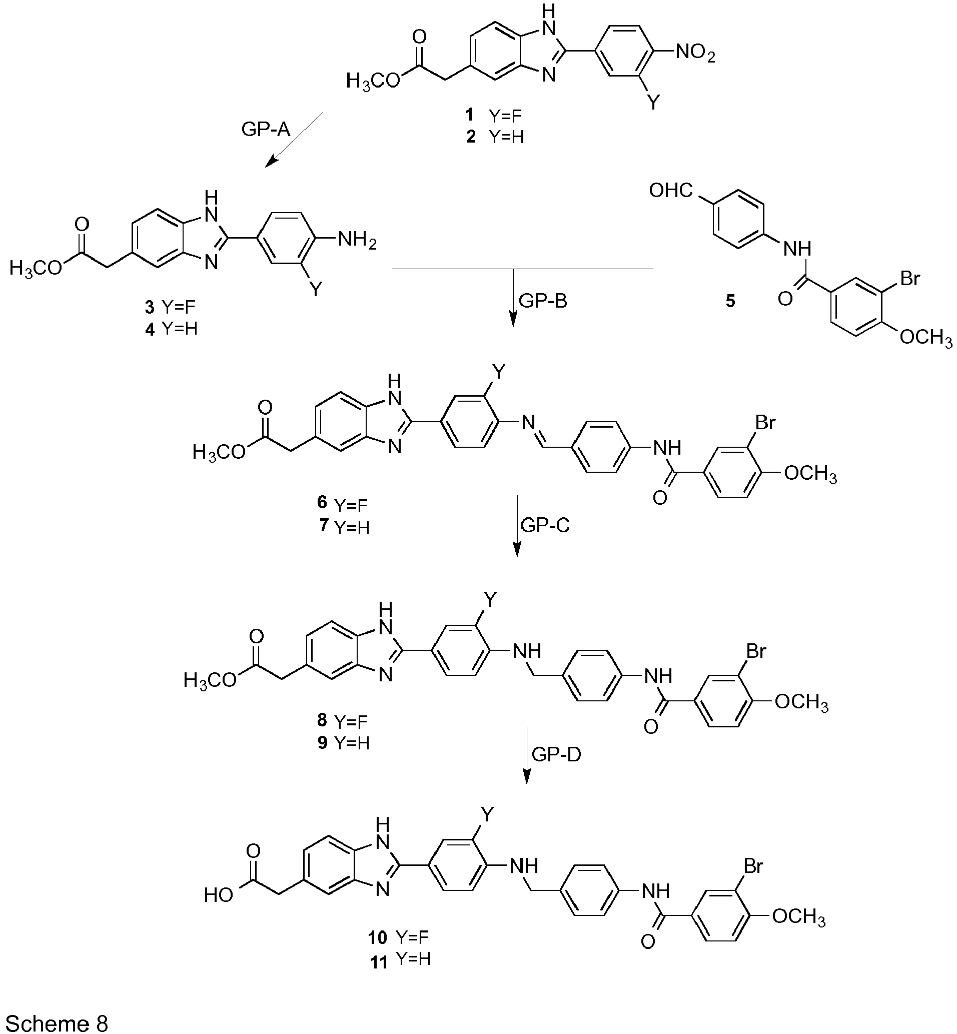

### General procedure A (GP-A) to obtain Amines (3) and (4).

Diethylamine (1 mmol), Pd/C 10% (10% w/w) and ammonium formate (10 mmol) were added to a well-stirred solution of the appropriate nitro derivative (1 mmol) in ethyl acetate. The mixture was refluxed for 1 h under nitrogen atmosphere. Then the reaction was quenched with water, cooled at room temperature, filtered on celite and washed with ethyl acetate. The organic layer was evaporated (or reduced to a small volume for compound 3 under vacuum and the raw material was extracted with ethyl acetate and water. The organic phase was separated, washed with brine, dried over sodium sulphate, filtered and evaporated under vacuum to afford the pure amine. For each compound amount of nitro derivative; volume of solvent; R_{f}; yield (%); melting point (°C); IR; ¹H NMR; MS (ESI) are reported.

### Methyl 2-(2-(4-amino-3-fluorophenyl)-1H-benzo[d]imidazol-5-yl)acetate (3).

Methyl 2-(2-(3-fluoro-4-nitrophenyl)-1*H*-benzo[*d*]imidazol-5-yl)acetate (1) (0.79 g, 2.4 mmol); ethyl acetate 160 mL; R_{f} (*n*-hexane/ethyl acetate 1:1): 0.15; 100% as a white solid; 247-250°C; IR v 3347 (NH), 3147 (NH₂), 1720 (C=O ester) cm⁻¹; ¹H NMR (dimethylsulfoxide *d₆*) *δ* 3.61 (s, 3H, OCH₃), 3.73-3.76 (m, 2H, CH₂ acetic), 5.67 (s, 2H, NH₂), 6.83-7.76 (m, 6H, Ar), 12.51 (bs, 1H, benzimidazole); MS: m/z (ESI) 300.2 [M+H]⁺.

### Methyl 2-(2-(4-aminophenyl)-1H-benzo[d]imidazol-5-yl)acetate (4).

Methyl 2-(2-(4-nitrophenyl)-1*H*-benzo[*d*]imidazol-5-yl)acetate (2) (1.5 g, 4.8 mmol); anhydrous methanol 50 mL; R_{f} (ethyl acetate): 0.24; 70% as a pink solid; 213-215°C; IR v 3216 (NH), 3141 (NH₂), 1716 (C=O ester) cm⁻¹; ¹H NMR (dimethylsulfoxide *d₆*) *δ* 3.61 (s, 3H, OCH₃), 3.73-3.75 (m, 2H, CH₂ acetic), 5.59 (s, 2H, NH₂), 6.64-7.82 (m, 7H, Ar), 12.37 (bs, 1H, benzimidazole); MS: m/z (ESI) 282.4 [M+H]⁺.

### General procedure B (GP-B) to obtain imine derivatives (6) and (7).

A well-stirred suspension of the proper amine (1 mmol), 3-bromo-N-(4-formylphenyl)-4-methoxybenzamide (5) (1 mmol) and p-toluenesulphonic acid monohydrate (0.01 mmol) in toluene (20 mL) was stirred at 150° C for 5-8 h. Water which formed was removed by the means of Dean-Stark apparatus. The yellow solid that formed was filtered, washed with warm toluene and hexane and dried under IR lamp to afford the pure imine derivative. For each compound amount of amino derivative; R_{f}; yield (%); melting point (°C); IR; ¹H NMR; MS (ESI) are reported.

### Methyl 2-(2-(4-((4-(3-bromo-4-methoxybenzamido)benzylidene)amino)-3-fluorophenyl)-1 H-benzo[d]imidazol-5-yl)acetate (6).

Methyl 2-(2-(4-amino-3-fluorophenyl)-1*H*-benzo[*d*]imidazol-5-yl)acetate (3) (0.74 g, 2.5 mmol); R_{f} (chloroform/methanol 8.5:1.5): 0.78; 72% as a yellow solid; 230-232°C; IR *v* 3329 (NH), 1716 (C=O ester), 1655 (C=O amide), 1634 (C=N) cm⁻¹; ¹H NMR (dimethylsulfoxide *d₆*) *δ* 3.63 (s, 3H, COOCH₃), 3.78-3.81 (m, 2H, CH₂ acetic), 3.95 (s, 3H, OCH₃), 7.09-8.28 (m, 13H, Ar), 8.71 (s, 1H, H imine), 10.49 (s, 1H, amide), 12.92 (bs, 1H, benzimidazole); MS: m/z (ESI) 615.5 [M+H]⁺.

### Methyl 2-(2-(4-((4-(3-bromo-4-methoxybenzamido)benzylidene)amino)phenyl)-1H-benzo[d]imidazol-5-yl)acetate (7).

Methyl 2-(2-(4-aminophenyl)-1*H*-benzo[*d*]imidazol-5-yl)acetate (4) (0.93 g, 3.31 mmol); R_{f} (chloroform/methanol 8.5:1.5): 0.68; 82% as a yellow solid; 221-224°C; IR *v* 3267 (NH), 1736 (C=O ester), 1661 (C=O amide), 1627 (C=N) cm⁻¹; ¹H NMR (dimethylsulfoxide *d₆*) δ 3.63 (s, 3H, COOCH₃), 3.79 (s, 2H, CH₂ acetic), 3.95 (s, 3H, OCH₃), 7.09-8.27 (m, 14H, Ar), 8.67 (s, 1H, H imine), 10.46 (s, 1H, amide), 12.88 (bs, 1H, benzimidazole) MS: m/z (ESI) 597.4 [M+H]⁺.

### General procedure C (GP-C) to obtain amines (8) and (9).

Sodium borohydride (2.58 mmol) was added into a well-stirred solution of the appropriate imine (1 mmol) refrigerated in an ice-bath in anhydrous tetrahydrofuran. The reaction, periodically checked by ¹HNMR, was stirred at room temperature under argon atmosphere and further amounts of sodium borohydride were added portionwise within 15-24 hours, in order to induce completion of reaction. The organic phase was removed under vacuum and the raw material was treated with water. The solid which formed was filtered, washed with water, petroleum ether and dried under IR lamp to afford the pure benzylamino derivative. For each compound amount of imine derivative; anhydrous tetrahydrofuran; additional amounts of sodium borohydride and time of addition; R_{f}; yield (%); melting point (°C); recrystallization solvent; IR; ¹H NMR; MS (ESI) are reported.

### Methyl 2-(2-(4-((4-(3-bromo-4-methoxybenzamido)benzyl)amino)-3-fluorophenyl)-1H-benzo[d]imidazol-5-yl)acetate (8).

Compound (6) (1.06 g, 1.7 mmol); 50 mL; 0.2 g (5.3 mmol) in 24 h; R_{f} (ethyl acetate): 0.7; 100% as a white solid; 200-202°C; washed with diethyl ether; IR v 3254 (NH), 1722 (C=O ester), 1625 (C=O amide) cm⁻¹; ¹H NMR (dimethylsulfoxide *d₆*) *δ* 3.61 (s, 3H, COOCH₃), 3.74 (s, 2H, CH₂ acetic), 3.93 (s, 3H, OCH₃), 4.40 (bd, 2H, CH₂benzylamine), 6.71 (bt, 1H, NH benzylamine), 7.00-8.21 (m, 13H, Ar), 10.16 (s, 1H, amide), 12.52 (bs, 1H, benzimidazole); MS: m/z (ESI) 617.4 [M+H]⁺.

### Methyl 2-(2-(4-((4-(3-bromo-4-methoxybenzamido)benzyl)amino)phenyl)-1H-benzo[d]imidazol-5-yl)acetate (9).

Compound (7) (0.15 g, 0.25 mmol); 7 mL; 0.1 g (2.64 mmol) in 15 h; R_{f} (ethyl acetate): 0.68; 100% as a white solid; 123-126°C; washed with diethyl ether; IR v 3329 (NH₂), 1720 (C=O ester), 1649 (C=O amide) cm⁻¹; ¹H NMR (dimethylsulfoxide *d₆*) δ 3.60 (s, 3H, COOCH₃), 3.72-3.75 (m, 2H, CH₂ acetic), 3.93 (s, 3H, OCH₃), 4.33 (bd, 2H, CH₂benzylamine), 6.62 (bt, 1H, NH benzylamine), 6.69-8.22 (m, 14H, Ar), 10.17 (s, 1H, amide), 12.38 (bs, 1H, benzimidazole); MS: m/z (ESI) 599.1 [M+H]⁺.

### General procedure D (GP-D) to obtain Acetic Acid derivatives (10) and (11).

A solution of LiOH (2.5 mmol) in distilled water was added to a solution of the appropriate ester (1 mmol) in tetrahydrofuran (fivefold of the water amount) and the reaction was stirred vigorously overnight. The organic phase was removed under vacuum and the resulting suspension was acidified with 1N HCI until pH 4-5 was obtained. The solid which formed was collected by filtration, then washed with water and dried under IR lamp to afford pure acids. For each compound amount of ester derivative; tetrahydrofuran; R_{f}; yield (%); melting point (°C); recrystallization solvent; IR; ¹H NMR are reported.

### 2-(2-(4-((4-(3-bromo-4-methoxybenzamido)benzyl)amino)-3-fluorophenyl)-1H-benzo[d]imidazol-5-yl)acetic acid (10) (SST0867AA1).

Compound (8) (1.06 g, 1.7 mmol); 20 mL; R_{f} (ethyl acetate/methanol 3:2): 0.32; 100% as a yellow solid; 180-183°C; isopropanol; IR v 3299 (NH benzylamine), 2937 (OH), 1722 (C=O acid) cm⁻¹;¹H NMR (dimethylsulfoxide *d₆*) δ 3.71 (s, 2H, CH₂ acetic), 3.93 (s, 3H, OCH₃), 4.45 (bd, 2H, CH₂benzylamine), 6.80 (bt, 1H, NH benzylamine), 7.03-8.21 (m, 13H, Ar), 10.18 (s, 1H, amide), 12.34 (bs, 1H, benzimidazole), 14.00 (bs, 1H, OH); MS: m/z (ESI) 603.3 [M+H]⁺.

### 2-(2-(4-((4-(3-bromo-4-methoxybenzamido)benzyl)amino)-phenyl)-1H-benzo[d]imidazol-5-yl)acetic acid (11). SST0914AA1

Compound (9) (0.13 g, 0.22 mmol); 3 mL; R_{f} (ethyl acetate:methanol 4:1): 0.18; 82% as a yellow solid; 283-286°C; isopropanol; IR v 3285 (NH benzylamine), 2940 (OH), 1711 (C=O acid) cm⁻¹; ¹H NMR (dimethylsulfoxide *d₆*) δ 3.72 (s, 2H, CH₂acetic), 3.93 (s, 3H, OCH₃), 4.37 (bd, 2H, CH₂benzylamine), 6.79 (m, 2H, NH benzylamine and 1H Ar), 7.23-8.22 (m, 13H, Ar), 10.19 (s, 1H, amide), 12.35 (bs, 1H, benzimidazole), 14.00 (bs, 1H, OH); MS: m/z (ESI) 585.5 [M+H]⁺.

### Example 2

### Compounds 15, 16, 17

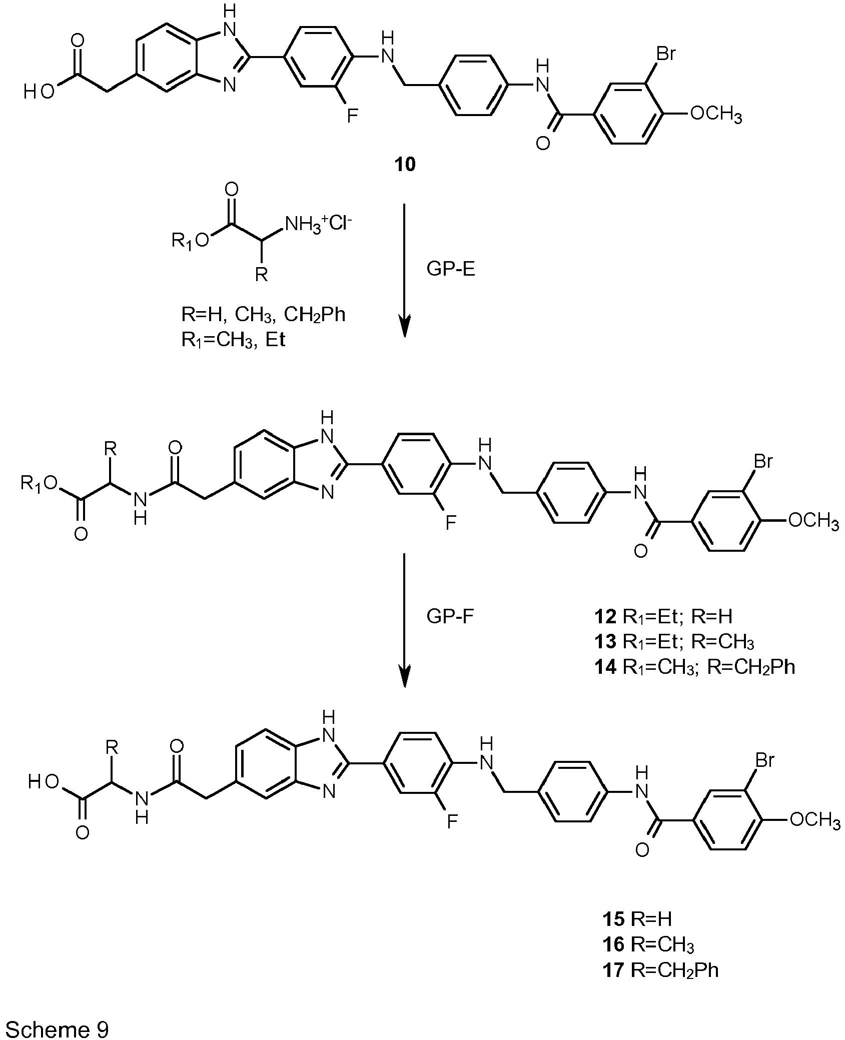

### General procedure E (GP-E) to obtain Amide derivatives (12), (13) and (14).

Amino ester hydrochloride (1.2 mmol), 1-ethyl-3-(3-dimethylaminopropyl)carbodiimide (EDCI), (1.2 mmol), 4-dimethylaminopyridine (DMAP) (1.2 mmol) and N,N-Diisopropylethylamine (DIPEA) (2.4 mmol) were added to a well-stirred solution of acid derivative (10) (1 mmol) in a anhydrous mixture of tetrahydrofuran/N,N-dimethylformamide. The mixture was stirred at room temperature overnight under argon atmosphere. The organic phase was removed under vacuum and the crude was treated with water. The solid which formed was filtered, washed with water and dried under IR lamp to afford the pure amide derivatives. For each compound amount of amino ester hydrochloride; tetrahydrofuran/N,N-dimethylformamide volumes; R_{f}; yield (%); melting point (°C); recrystallization solvent; IR; ¹H NMR; purity; and MS (ESI) are reported.

### Ethyl 2-(2-(2-(4-((4-(3-bromo-4-methoxybenzamido)benzyl)amino)-3-fluorophenyl)-1H-benzo[d]imidazol-5-yl)acetamido)acetate (12).

Glycine ethyl ester hydrochloride (56 mg, 0.40 mmol); 35 mL/6 mL; 0.63 (ethyl acetate/methanol 4:1); 80% as a yellow solid; 160-163°C; ethyl acetate; IR v 3273 (NH), 1736 (C=O), 1626, 1600 (C=O amide) cm⁻¹; ¹H NMR (dimethylsulfoxide *d₆*) *δ* 1.16 (t, 3H, CH₃CH₂O, J = 4 Hz), 3.54-3.56 (m, 2H, CH₂ acetic), 3.81 (d, 2H, CH₂Gly, J = 4 Hz), 3.93 (s, 3H, OCH₃), 4.06 (q, 2H, CH₃CH₂O, J = 4Hz), 4.41 (bd, 2H, CH₂ benzylamine), 6.71 (bt, 1H, NH benzylamine), 7.02-8.43 (m, 13H, Ar), 10.17 (s, 1H, amide), 12.46 (bs, 1H, benzimidazole); MS: m/z (ESI) 688.4 [M+H]⁺.

### Ethyl 2-(2-(2-(4-((4-(3-bromo-4-methoxybenzamido)benzyl)amino)-3-fluorophenyl)-1H-benzo[d]imidazol-5-yl)acetamido)propanoate (13).

L-Alanine ethyl ester hydrochloride (0.092 g, 0.60 mmol); 50 mL/3 mL; R_{f} (ethyl acetate/methanol 4:1) 0.55; 88.3% as a white-yellow solid; 145-148°C; ethyl acetate; IR v 3276 (NH), 1732 (C=O ester), 1647, 1626 (C=O amide) cm⁻¹; ¹H NMR (dimethylsulfoxide *d₆*) *δ* 1.14 (t, 3H, CH₃CH₂O, J = 4 Hz), 1.28 (d, 3H, CH₃ Ala, J = 8Hz), 3.51-3.53 (m, 2H, CH₂ acetic), 3.93 (s, 3H, OCH₃), 4.04 (q, 2H,CH₃CH₂O, J = 4Hz), 4.19-4.26 (m, 1H, CHAla), 4.42 (bd, 2H, CH₂ benzylamine), 6.70 (bt, 1H, NH benzylamine), 7.01-8.49 (m, 13H, Ar), 10.16 (s, 1H, amide), 12.45 (bs, 1H, benzimidazole); MS: m/z (ESI) 702.5 [M+H]⁺.

### Methyl 2-(2-(2-(4-((4-(3-bromo-4-methoxybenzamido)benzyl)amino)-3-fluorophenyl)-1H-benzo[d]imidazol-5-yl)acetamido)-3-phenylpropanoate (14).

L-Phenylalanine methyl ester hydrochloride (0.13 g, 0.60 mmol); 50 mL/3 mL; R_{f} (ethyl acetate/methanol 4:1): 0.56; 83% as a white-yellow solid; 154-156°C; ethyl acetate; IR v 3273 (NH), 1738 (C=O), 1648, 1626 (C=O amide) cm⁻¹; ¹H NMR (dimethylsulfoxide *d₆*) *δ* 2.90-2.95 (m, 1H, CH₂PhAla), 3.00-3.05 (m, 1H, CH₂PhAla), 3.49-3.50 (m, 2H, CH₂ acetic), 3.58 (s, 2H, CH₃O ester), 3.94 (s, 3H, OCH₃), 4.41-4.49 (m, 3H, CH₂ benzylamine and CH PhAla), 6.71 (bt, 1H, NH benzylamine) 6.90-8.51 (m, 18H, Ar), 10.17 (s, 1H, amide), 12.47 (bs, 1H, benzimidazole); MS: m/z (ESI) 764.4 [M+H]⁺.

### General procedure F (GP-F) to obtain acid derivatives (15), (16) and (17).

A solution of lithium hydroxide (2.5 mmol) in distilled water was added to a solution of the appropriate ester (1 mmol) in tetrahydrofuran (fivefold the water amount) and the reaction was stirred vigorously overnight. The organic phase was removed under vacuum and the resulting suspension was acidified with 1N HCI until pH 4-5 was obtained. The solid formed was collected by filtration, then washed with water and dried under IR lamp to afford pure acids. For each compound amount of ester; tetrahydrofuran volume; R_{f}; yield (%); melting point (°C); recrystallization solvent; IR; ¹H NMR; purity; and MS (ESI) are reported.

### 2-(2-(2-(4-((4-(3-bromo-4-methoxybenzamido)benzyl)amino)-3-fluorophenyl)-1H-benzo[d]imidazol-5-yl)acetamido)acetic acid (15) (SST0871AA1).

Compound (12) (0.15 g, 0.22 mmol); 21 mL; 0.18 (ethyl acetate/methanol 3:2); 100% as an orange solid; 187-189°C; IR *v* 3314 (NH benzylamine), 2842 (OH), 1731 (C=O acid), 1618, 1600 (C=O amide) cm⁻¹; ¹H NMR (dimethylsulfoxide *d₆*) *δ* 3.64 (s, 2H, CH₂ acetic), 3.76 (bd, 2H, CH₂ Gly), 3.93(s, 3H, OCH₃), 4.46 (bd, 2H, CH₂ benzylamine), 6.84 (bt, 1H, NH benzylamine), 7.23-8.42 (m, 13H, Ar), 10.18 (s, 1H, amide), 12.55 (bs, 1H, benzimidazole), 14.31 (bs, 1H, OH); 99.46%; MS: m/z (ESI) 660.1 [M+H]⁺.

### ((2S)-2-(2-(4-((4-(3-bromo-4-methoxybenzamido)benzyl)amino)-3-fluorophenyl)-1H-benzo[d]imidazol-5-yl)acetyl)-alanine (16) (SST0868AA1).

Compound (13) (0.29 g, 0.41 mmol); 10 mL; R_{f} (ethyl acetate/methanol 3:2): 0.23; 98% as an orange solid; 175-178°C; isopropanol; IR v 3280 (NH benzylamine), 2939 (OH), 1724 (C=O acid), 1619 (C=O amide) cm⁻¹; ¹H NMR (dimethylsulfoxide *d₆*) *δ* 1.27 (d, 3H, CH₃ Ala, J = 4 Hz), 3.62 (s, 2H, CH₂ acetic), 3.93 (s, 3H, OCH₃), 4.17-4.23 (m, 1H, CH Ala), 4.47 (bd, 2H, CH₂ benzylamine), 6.84 (t, 1H, NH benzylamine, J = 8 Hz), 7.23-8.49 (m, 13H, Ar), 10.19 (s, 1H, amide), 12.52 (bs, 1H, benzimidazole), 14.55 (br s, 1H, OH); 99.99%; MS: m/z (ESI) 674.1 [M+H]⁺.

### ((2S)-2-(2-(4-((4-(3-bromo-4-methoxybenzamido)benzyl)amino)-3-fluorophenyl)-1H-benzo[d]imidazol-5-yl)acetyl)-phenylalanine (17) (SST0869AA1).

Compound (14) (0.3 g, 0.39 mmol); 10 mL; R_{f} (ethyl acetate/methanol 3:2): 0.29; 92% as a yellow solid; 179-181°C; IR v 3300 (NH benzylamine), 2938 (OH), 1731 (C=O acid), 1620, 1600 (C=O amide) cm⁻¹; ¹H NMR (dimethylsulfoxide *d₆*) *δ* 2.84-2.90 (m, 1H, CH₂PhAla), 3.02-3.06 (m, 1H, CH₂PhAla), 3.51-3.52 (m, 2H, CH₂ acetic), 3.93 (s, 3H, OCH₃), 4.38-4.43 (m, 3H, CH PhAla and CH₂ benzylamine), 6.75 (t, 1H, NH benzylamine, J = 8 Hz), 6.87-8.35 (m, 13H, Ar), 10.16 (s, 1H, amide), 12.72 (bs, 1H, benzimidazole), 14.31 (bs, 1H, OH); 99.99%; MS: m/z (ESI) 750.0 [M+H]⁺.

### Example 3

### Compound 19

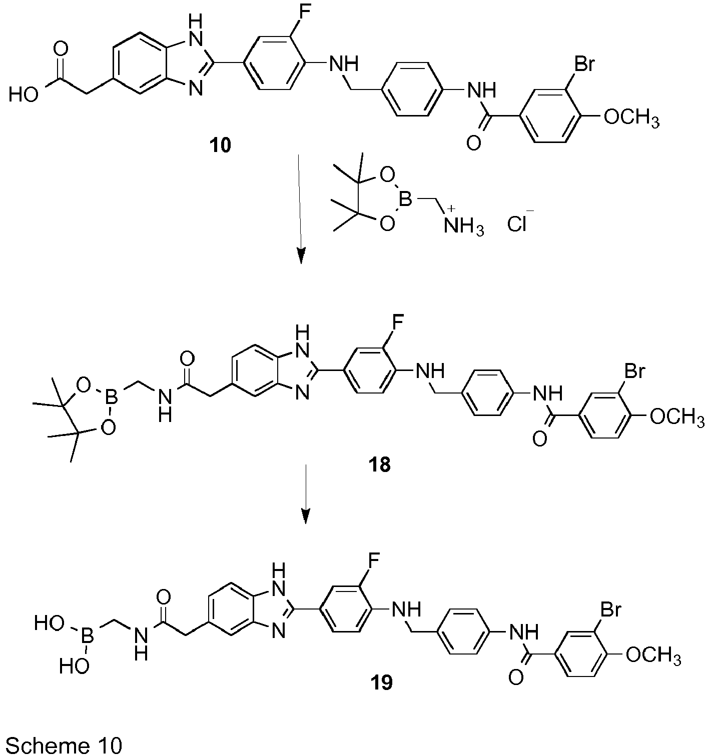

### 3-Bromo-N-(4-(((2-fluoro-4-(5-(2-oxo-2-(((4,4,5,5-tetramethyl-1,3,2-dioxaborolan-2-yl)methyl)amino)ethyl)-1H-benzo[d]imidazol-2-yl)phenyl)amino)methyl)phenyl)-4-methoxybenzamide (18).

Compound 10 (0.2 g, 0.33 mmol), (4,4,5,5-tetramethyl-1,3,2-dioxaborolan-2-yl)methanaminium chloride (0.77 g, 0.34 mmol), and O-(benzotriazol-1-yl)-N,N,N',N'-tetramethyluronium tetrafluoroborate (TBTU) (0.15 g, 0.45 mmol) were solubilized in anhydrous tetrahydrofuran (30 mL), and the mixture was cooled to -80° C while stirring. After that a solution of N,N-diisopropylethylamine (DIPEA) (0.15 g, 1.20 mmol, 0.21 mL) in anhydrous tetrahydrofuran (4 mL) was added dropwise in 2 h into reaction mixture while stirring, maintaining the temperature at -80°C. The mixture was further stirred for 1.5 h, and then slowly heated to room temperature. The solvent was removed under vacuum and the crude was treated with distilled water, the solid which formed was filtered, washed with water and dried under IR lamp to afford the pure product (0.20 g, 82%) as a yellow solid. R_{f} (ethyl acetate/methanol 9:1): 0.8; 200-201 °C; washed with diethyl ether; IR v 3295 (NH), 1622 (C=O amide), 1600 (C=O amide) cm⁻¹. ¹H NMR (dimethylsulfoxide d*₆*) δ 1.06 (s, 12H, CH₃C), 2.13 (br d, 1H, CH₂B), 3.50 (s, 1H, ArCH₂CO), 3.70 (br d, 1H, CH₂B), 3.94 (s, 3H, OCH₃), 4.42-4.43 (d, 2H, ArCH₂NH J = 4 Hz), 6.72-6.81 (m, 2H, benzene H, and Ar-NHCH₂), 7.24 (t, 2H, benzimidazole C6-H and C4-H, J = 8 Hz), 7.36 (d, 2H, benzene H, J = 8 Hz), 7.59 (s, 1H, benzene H), 7.62 (d, 1H, benzimidazole C7-H, J = 8 Hz), 7.70-7.80 (m, 4H, benzene H), 8.00 (d, 1H, benzene H, J = 8 Hz), 8.22 (s, 1H, benzene H), 9.24 (br t, 1H, CH₂NHCO), 10.17(s, 1H, NHCO), 12.94 (br s, 1H, imidazole H); 95.62%; MS: m/z (ESI) 764.3 [M+Na]⁺.

### ((2-(2-(4-((4-(3-Bromo-4-methoxybenzamido)benzyl)amino)-3-fluorophenyl)-1H-benzo[d]imidazol-5-yl)acetamido)methyl)boronic acid (19) (SST0873AA1).

A solution of aqueous ammonium acetate (3.0 mL, 0.1 N) and sodium periodate (0.02 g, 0.29 mmol) was added into a solution of compound (18) (0.10 g, 0.13 mmol) in tetrahydrofuran (5 mL). The mixture was vigorously stirred at room temperature overnight. The organic solvent was removed under vacuum and the resulting solid was collected by filtration, washed with water, dried under IR lamp and then washed with diethyl ether to afford compound (19) (SST0873AA1) as an orange solid (0.03 g, 35%). R_{f} (ethyl acetate:methanol 9:1): 0.43; 241-242°C; washed with diethyl ether; IR *v* 3278 (OH), 2944 (NH), 1621 (C=O amide), 1599 (C=O amide)cm⁻¹. ¹H NMR (dimethyl sulfoxide d*₆*) δ 3.49 (s, 2H, ArCH₂CO), 3.93 (s, 3H, OCH₃), 4.42 (br d, 2H, ArCH₂NH), 4.50 (br d, 2H, CH₂B), 6.71-6.76 (m, 1H, benzene H, and Ar-NHCH₂), 7.05-7.07 (m, 2H, benzimidazole C6-H and C4-H), 7.24-7.26 (d, 2H, benzene H, J = 8 Hz), 7.35-7.44 (m, 4H, benzene H and benzimidazole C7-H), 7.76-7.83 (m, 3H, benzene H), 8.05 (d, 1H, benzene H, J = 8 Hz), 8.22 (s, 1H, benzene H), 8.60 (br t, 1H, CH₂NHCO), 8.83 (br s, 2H, OH), 10.17 (s, 1H, NHCO); 98.43%.

### Example 4

### Compound 22

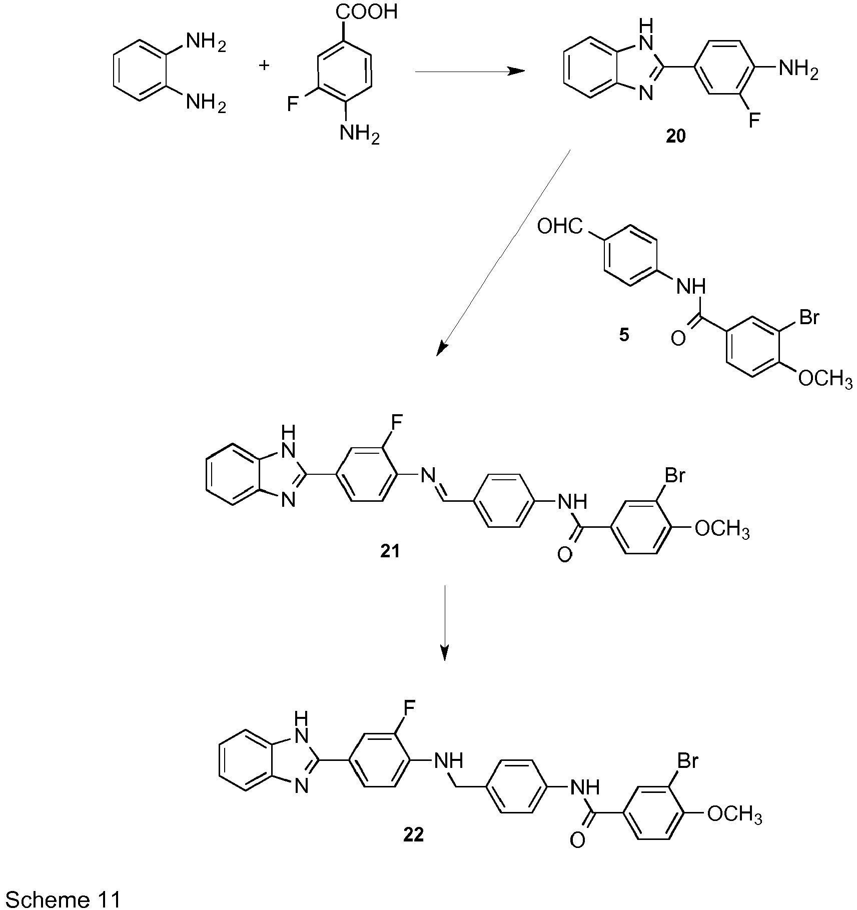

### (4-amino-3-fluorophenyl)-1H-benzo[d]imidazole (20).

1,2-Phenylendiamine (6.5 g, 60 mmol), 3-fluoro-4-aminobenzoic acid (7.9 g, 51 mmol) and polyphosphoric acid (25 g) were put in a round flask and stirred a 220 °C for 5 hours (a dark oily mixture formed almost immediately). After cooling, potassium carbonate (10%, 400 mL) was added to this dark oily crude; the lump formed, was neutralized to pH=7 with saturated solution of NaHCO₃ and, after lump turned to suspension, the solid was recovered by filtration. The crude product was washed with hot water (50-70 °C) till the water was colorless and, after crystallization by ethyl acetate (1 L) and filtration on charcoal compound (20) (7 g) was obtained as a pure light brown solid. Yield 60%. ¹H-NMR: δ 12.56 (bs, 1H), 7.78 (dd, *J₁* = 12.7 Hz, *J₂ =* 1.5 Hz, 1H), 7.72 (d, *J* = 8.3 Hz, 1H), 7.50 (bs, 2H), 7.13 (m, 2H), 6.86 (t, *J* = 8.8 Hz, 1H), 5.66 (s, 2H). MS: m/z (ESI) 228.0 [M+H]⁺, 226.0 [M-H]⁻.

### 2-(4-(4-(3-bromo-4-methoxybenzamido)benzylidene)amino)-3-fluorophenyl)-1H-benzo[d]imidazole (21).

Compound (20) (50 mg, 0.22 mmol) and compound (5) (73 mg, 0.22 mmol) were suspended in ethanol (5 mL) with few drops of acetic acid and the mixture was warmed at 70 °C. After few minutes starting materials were completely dissolved and a new precipitate formed after about 20 minutes. TLC and MS flow injection confirmed completely conversion of starting materials. The mixture was cooled at room temperature and filtered and the solid was washed with ethanol (2 mL) to give 72 mg of compound (21) as a quite pure yellow solid that was converted directly in the next step without further purification. Yield 60%. MS: m/z (ESI) 543.2 [M+H]⁺, 565.1 [M+Na]⁺.

### 2-(4-(4-(3-bromo-4-methoxybenzamido)benzyl)amino)-3-fluorophenyl)-1H-benzo[d]imidazole (22) (SST0623AA1).

Compound (21) (70 mg, 0.13 mmol) was dissolved in N,N-dimethylformamide and sodium borohydride (8 mg, 0.21 mmol) was added under stirring at room temperature. After 2 hours starting material was totally converted. The mixture pH was neutralized to 7 by HCI 3N addition; after concentration under reduced pressure, the crude product was purified by silica gel chromatography (eluent dichloromethane/methanol 97:3) to give about 50 mg of compound (22) (SST0623AA1) as a pure light yellow solid. Yield 70%. 1 H-NMR: δ 10.19 (s, 1H), 8.22 (s, 1H), 8.01 (d, *J* = 7.8 Hz, 1H), 7.93 (d, *J* = 12.2 Hz, 1H), 7.80 (d, *J* = 7.3 Hz, 1H), 7.72 (d, *J* = 6.8 Hz, 2H), 7.93 (bs, 2H), 7.37 (d, *J* = 6.8 Hz, 2H), 7.30 (bs, 2H), 7.25 (d, *J* = 7.8 Hz, 1H), 7.08 (bs, 1H), 6.81 (t, *J* = 7.0 Hz, 1H), 4.45 (s, 2H), 3.94 (s, 3H). MS: m/z (ESI) 545.0 [M+H]⁺, 567.0 [M+Na]⁺, 543.0 [M-H]⁻.

### Example 5

### Compounds 29, 30, 37, 38, 39, 40, 41

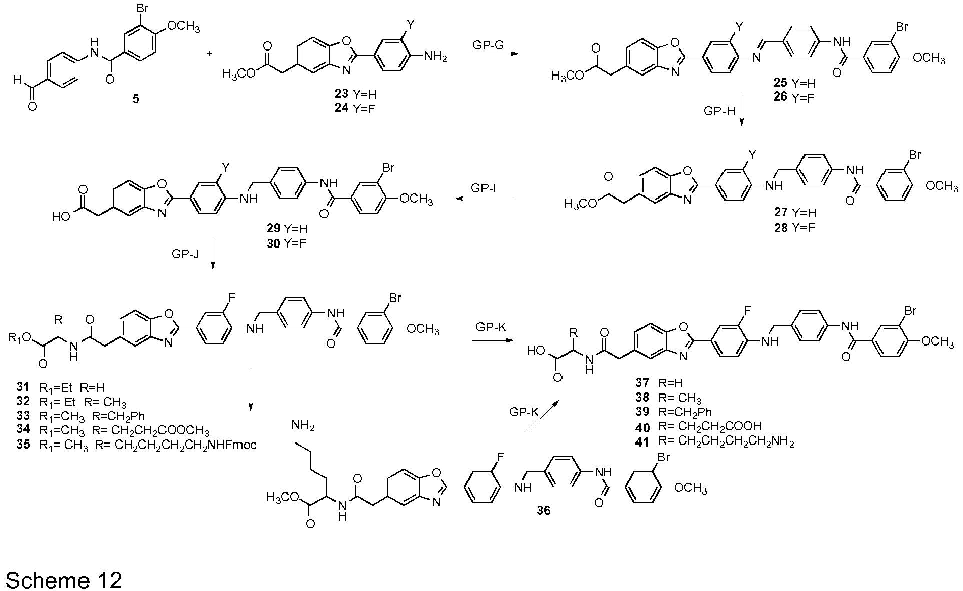

### General procedure G (GP-G) to obtain imine derivatives (25) and (26).

A well-stirred suspension of the opportune amine (1 mmol), 3-bromo-N-(4-formylphenyl)-4-methoxybenzamide (5) (1 mmol) and *p*-toluenesulfonic acid monohydrate (0.01 mmol) in toluene (27 mL) was stirred at 150° C for 5-8 h. Water which formed was removed by the means of Dean-Stark apparatus. The yellow solid that formed was filtered, washed with warm toluene and hexane and dried under IR lamp to afford the pure imine derivative. For each compound amount of amine; R_{f}; yield (%); melting point (°C); IR;¹H NMR and MS (ESI) are reported.

### Methyl (E)-2-(2-(4-((4-(3-bromo-4-methoxybenzamido)benzylidene)amino)phenyl)benzo[d]oxazol-5-yl)acetate (25).

Compound (23) (0.7 g, 2.48 mmol); R_{f} (*n*-hexane/ethyl acetate 1:1): 0.4; 70% as a yellow solid; 235 °C; IR v 1731 (C=O ester), 1635 (N=CH imine) cm⁻¹; ¹H NMR (dimethylsulfoxide d*₆*) δ 3.65 (s, 3H, CH₃), 3.85 (s, 2H, CH₂), 3.96 (s, 3H, OCH₃), 7.25-7.35 (m, 2H, benzene H and benzoxazole C6-H), 7.47 (d, 2H, benzene H, J = 8 Hz), 7.70-7.75 (m, 2 H, benzoxazole C4-H and benzene H), 8.00-8.10 (m, 5H, benzene H), 8.23-8.27 (m, 3H, benzene H), 8.66 (s, 1H, N=CH), 10.47 (s, 1H, NHCO); 95%; MS: m/z (ESI) 598.2 [M+H]⁺.

### Methyl (E)-2-(2-(4-((4-(3-bromo-4-methoxybenzamido)benzylidene)amino)-3-fluorophenyl)benzo[d]oxazol-5-yl)acetate (26).

Compound (24) (0.3 g, 1 mmol); R_{f} (*n*-hexane/ethyl acetate 1:1): 0.6; 77% as a yellow solid; 238 °C; tetrahydrofuran; IR v 1731 (C=O ester), 1635 (N=CH imine) cm⁻¹; ¹H NMR (dimethylsulfoxide d*₆*) δ 3.65 (s, 3H, CH₃,), 3.86 (s, 2H, CH₂), 3.96 (s, 3H, OCH₃), 7.29 (d, 1H, benzene H, J = 8 Hz), 7.36 (d, 1H, benzene H, J = 8 Hz), 7.55 (t, 1H, benzene H, J = 8 Hz), 7.73-7.77 (m, 2 H, benzoxazole C4-H and C6-H), 8.00-8.10 (m, 6H, benzene H and benzoxazole C7-H,), 8.28 (br d, 1H, benzene H), 8.71 (s, 1H, NCH), 10.51 (s, 1H, NHCO); 95.62%; MS: m/z (ESI) 616.4 [M+H]⁺.

### General procedure H (GP-H) to obtain benzylamine derivatives (27) and (28).

Sodium borohydride (2.58 mmol) was added into a well-stirred solution of the appropriate imine (1 mmol) in 3:1 dichloromethane/methanol, cooled at 0-4 °C. The mixture was stirred at room temperature under argon atmosphere for the appropriate time to complete the reaction. The organic phase was removed under vacuum and the raw material was treated with water. The solid which formed was filtered, washed with water, petroleum ether and dried under IR lamp to afford the pure benzylamine derivatives. For each compound amount of imine; volume of solvent; time of reaction; R_{f}; yield (%); melting point (°C); IR; ¹H NMR and MS (ESI) are reported.

### Methyl 2-(2-(4-((4-(3-bromo-4-methoxybenzamido)benzyl)amino)phenyl)benzo[d]oxazol-5-yl)acetate (27).

Compound (25) (0.5 g, 0.83 mmol); 40 mL; R_{f} (n-hexane/ethyl acetate 1:1): 0.62; 60% as a yellow solid; 230-231 °C; IR v 3374 (NH) 1733 (C=O ester), 1648 (C=O amine) cm⁻¹; ¹H NMR (dimethylsulfoxide d*₆*) δ 3.63 (s, 3H, COOCH₃), 3.79 (s, 2H, OCOCH₂), 3.94 (s, 3H, OCH₃), 4.36 (br d, 2H, NHCH₂), 6.75 (d, 2H, benzene H, J = 8 Hz), 7.13 (br t, 1H, NHCH₂), 7.20 (d, 1H, benzoxazole C6-H, J = 8 Hz), 7.25 (d, 1H, benzene H, J = 8 Hz), 7.35 (d, 2H, benzene H, J = 8 Hz), 7.55 (s, 1H, benzoxazole C4-H), 7.60 (d, 1H, benzoxazole C7-H, J = 8 Hz), 7.72 (d, 2H, benzene H, J = 8 Hz), 7.89 (d, 2H, benzene H, J = 8 Hz), 8.01 (d, 1H, benzene H, J = 8 Hz), 8.22 (s, 1H, benzene H), 10.18 (s, 1H, NHCO); 95.62%; MS: m/z (ESI) 600.2 [M+H]⁺.

### Methyl 2-(2-(4-((4-(3-bromo-4-methoxybenzamido)benzyl)amino)-3-fluorophenyl)benzo[d]oxazol-5-yl)acetate (28).

Compound (26) (0.48 g, 0.77 mmol); 20 mL; R_{f} (n-hexane/ethyl acetate 1:1): 0.5; 100% as a yellow solid; 238 °C; IR v 3375 (NH) 1732 (C=O ester), 1650 (C=O amine) cm⁻¹; ¹H NMR (dimethylsulfoxide d*₆*) δ 3.63 (s, 3H, COOCH₃), 3.81 (s, 2H, OCOCH₂), 3.93 (s, 3H, OCH₃), 4.44 (br d, 2H, NHCH₂), 6.76 (t, 1H, benzene H, J = 8 Hz), 7.13 (br t, 1H, NHCH₂), 7.24 (t, 2H, benzoxazole C6-H and C4-H, J = 8 Hz), 7.36 (d, 2H, benzene H, J = 8 Hz), 7.59 (s, 1H, benzoxazole C4-H), 7.63 (d, 1H, benzoxazole C7-H,, J = 8 Hz), 7.70-7.80 (m, 4H, benzene H), 8.00 (br d, 1H, benzene H), 8.22 (s, 1H, benzene H), 10.18 (s, 1H, NHCO); MS: m/z (ESI) 618.5 [M+H]⁺.

### General procedure I (GP-I) to obtain acid derivatives (29) and (30).

A solution of lithium hydroxide (2.5 mmol) in distilled water was added to a solution of the appropriate ester (1 mmol) in tetrahydrofuran (fivefold the water amount) and the reaction was vigorously stirred overnight. The organic phase was removed under vacuum and the resulting suspension was acidified with 1N HCI until pH 4-5 was obtained. The solid which formed was collected by filtration, then washed with water and dried under IR lamp to afford pure acids. For each compound amount of ester; tetrahydrofuran; R_{f}; yield (%); melting point (°C); recrystallization solvent; IR; ¹H NMR, purity (%); and MS (ESI) are reported.

### 2-(2-(4-((4-(3-bromo-4-methoxybenzamido)benzyl)amino)phenyl)benzo[d]oxazol-5-yl)acetic acid (29). (SST0915AA1)

Compound (27) (0.2 g, 0.33 mmol); 8.8 mL; R_{f} (ethyl acetate): 0.6; 88% as a yellow solid; 249-250 °C; washed with diethyl ether; IR v 3286 (OH), 1708 (C=O acid), 1636 (C=O amine) cm⁻¹; ¹H NMR (dimethylsulfoxide d*₆*) δ 3.60 (s, 2H, OCOCH₂), 3.94 (s, 3H, OCH₃), 4.36 (br d, 2H, NHCH₂), 6.75 (d, 2H, benzene H, J = 8 Hz), 7.11 (br t, 1H, NHCH₂), 7.18 (d, 1H, benzoxazole C6-H, J = 8 Hz), 7.25 (d, 1H, benzene H, J = 8 Hz), 7.35 (d, 2H, benzene H, J = 8 Hz), 7.52 (s, 1H, benzoxazole C4-H), 7.56 (d, 1H, benzoxazole C7-H, J = 8 Hz), 7.72 (d, 2H, benzene H, J = 8 Hz), 7.89 (d, 2H, benzene H, J = 8 Hz), 8.01 (d, 1H, benzene H, J = 8 Hz), 8.22 (s, 1H, benzene H), 10.19 (s, 1H, NHCO), 12.10 (br s, 1H, COOH); MS: m/z (ESI) 586.4 [M+H]⁺.

### 2-(2-(4-((4-(3-bromo-4-methoxybenzamido)benzyl)amino)-3-fluorophenyl)benzo[d]oxazol-5-yl)acetic acid (30) (SST0864AA1).

Compound (28) (0.48 g, 0.77 mmol); 17.7 mL; R_{f} (n-hexane/ethyl acetate 1:1): 0.5; 100% as a yellow solid; 238 °C; washed with diethyl ether; IR v 3375 (NH) 1732 (C=O ester), 1650 (C=O amine) cm⁻¹; ¹H NMR (dimethylsulfoxide d*₆*) δ 3.63 (s, 3H, COOCH₃), 3.81 (s, 2H, OCOCH₂), 3.93 (s, 3H, OCH₃), 4.44 (br d, 2H, NHCH₂), 6.76 (t, 1H, benzene H, J = 8 Hz), 7.13 (br t, 1H, NHCH₂), 7.24 (t, 2H, benzoxazole C6-H and C4-H, J = 8 Hz), 7.36 (d, 2H, benzene H, J = 8 Hz), 7.59 (s, 1H, benzoxazole C4-H), 7.63 (d, 1H, benzoxazole C7-H, J = 8 Hz), 7.70-7.80 (m, 4H, benzene H), 8.00 (br d, 1H, benzene H), 8.22 (s, 1H, benzene H), 10.18 (s, 1H, NHCO); MS: m/z (ESI) 604.1 [M+H]⁺.

### General procedure J (GP-J) to obtain amide derivatives (31), (32), (33), (34) and (35).

Amino ester hydrochloride (2.4 mmol), 1-ethyl-3-(3-dimethylaminopropyl)carbodiimide (EDCI) (2.4 mmol), 4-dimethylaminopyridine (DMAP) (2.4 mmol) and N,N-diisopropylethylamine (DIPEA) (4.8 mmol) were added into a well-stirred mixture of compound (30) (1 mmol) in anhydrous tetrahydrofuran. The reaction was stirred to room temperature overnight under argon atmosphere. The organic phase was removed under vacuum and the crude was treated with water. The solid which formed was filtered, washed with water and dried under IR lamp to afford the pure amide derivatives. For each compound amount of amino ester hydrochloride; tetrahydrofuran volume; R_{f}; yield (%); melting point (°C); IR; ¹H NMR; purity (%) and MS (ESI) are reported.

### Ethyl (2-(2-(4-((4-(3-bromo-4-methoxybenzamido)benzyl)amino)-3-fluorophenyl)benzo[d]oxazol-5-yl)acetyl)glycinate (31).

Glycine ethyl ester hydrochloride (0.06 g, 0.40 mmol); 50 mL; R_{f}(ethyl acetate): 0.63; 88% as an yellow solid; 200-201°C; IR: *v* 3054 (NH), 1728 (C=O ester), 1649 (C=O amide), 1598 (C=O amide) cm⁻¹; ¹H NMR (dimethylsulfoxide d*₆*) δ 1.17 (t, 3H, CH₂CH₃, J = 8 Hz), 3.60 (s, 2H, ArCH₂CO), 3.84 (d, 2H, NHCH₂CO, J = 4 Hz), 3.93 (s, 3H, OCH₃), 4.08 (q, 2H, CH₂CH₃, J = 8 Hz), 4.44 (br d, 2H, NHCH₂), 6.76 (t, 1H, benzene H, J = 8 Hz), 7.11 (br t, 1H, NHCH₂), 7.24 (t, 2H, benzoxazole C6-H and C4-H, J = 8 Hz), 7.36 (d, 2H, benzene H, J = 8 Hz), 7.59 (s, 1H, benzene H), 7.62 (d, 1H, benzoxazole C7-H, J = 8 Hz), 7.70-7.80 (m, 4H, benzene H), 8.00 (d, 1H, benzene H, J = 8 Hz), 8.22 (s, 1H, benzene H), 8.51 (t, 1H, NHCH₂CO, J = 4 Hz), 10.18 (s, 1H, NHCO); 95.62%; MS: m/z (ESI) 687.1 [M-H]⁻.

### Ethyl (2-(2-(4-((4-(3-bromo-4-methoxybenzamido)benzyl)amino)-3-fluorophenyl)benzo[d]oxazol-5-yl)acetyl)alaninate (32).

L-Alanine ethyl ester hydrochloride (0.09 g, 0.60 mmol); 50 mL; R_{f} (ethyl acetate/methanol 9:1): 0.66; 82% as a yellow solid; 235-238°C; IR *v* 3273 (NH), 1735 (C=O ester), 1673 (C=O amide), 1628 (C=O amide) cm⁻¹; ¹H NMR (dimethylsulfoxide d*₆*) δ 1.14 (t, 3H, CH₂CH₃, J = 8 Hz), 1.29 (br d, 3H, CH₃CH), 3.57 (s, 2H, ArCH₂CO), 3.93 (s, 3H, OCH₃), 4.05 (q, 2H, CH₂CH₃, J = 8 Hz), 4.23 (m, 1H, CH₃CH), 4.44 (br d, 2H, NHCH₂Ph), 6.76 (t, 1H, benzene H, J = 8 Hz), 7.11 (br t, 1H, NHCH₂), 7.24 (t, 2H, benzoxazole C6-H and C4-H, J = 8 Hz), 7.36 (d, 2H, benzene H, J = 8 Hz), 7.57 (s, 1H, benzene H), 7.61 (d, 1H, benzoxazole C7-H,, J = 8 Hz), 7.70-7.80 (m, 4H, benzene H), 8.00 (d, 1H, benzene H, J = 8 Hz), 8.22 (s, 1H, benzene H), 8.51 (br d, 1H, NHCHCH₃), 10.18 (s, 1H, NHCO); MS: m/z (ESI) 703.1 [M+H]⁺.

### Methyl (2-(2-(4-((4-(3-bromo-4-methoxybenzamido)benzyl)amino)-3-fluorophenyl)benzo[d]oxazol-5-yl)acetyl)phenylalaninate (33).

L-Phenylalanine methyl ester hydrochloride (0.13 g, 0.6 mmol); 50 mL; R_{f} (ethyl acetate/methanol 9:1): 0.87; 94% as a yellow solid; 162-164°C; IR *v* 3280 (NH), 1738 (C=O ester), 1644(C=O amide), 1623 (C=O amide) cm⁻¹; ¹H NMR (dimethylsulfoxide d*₆*) δ 2.99 (br d, 2H, CH₂CH), 3.53 (s, 2H, ArCH₂CO), 3.60 (s, 3H, COOCH₃), 3.93 (s, 3H, OCH₃), 4.44-4.50 (m, 1H, CH₂CH), 6.76 (t, 1H, benzene H, J = 8 Hz), 7.05-30 (m, 9H, NHCH₂ and PhCH₂), 7.36 (d, 1H, benzoxazole C6-H, J = 8 Hz), 7.49 (s, 1H, benzoxazole C4-H), 7.56 (s, 1H, benzoxazole C7-H, J = 8 Hz), 7.70-7.80 (m, 4H, benzene H), 8.00 (d, 1H, benzene H, J = 8 Hz), 8.22 (s, 1H, benzene H), 8.41 (br d, 1H, COCHNH), 10.18 (s, 1H, NHCO); MS: m/z (ESI) 765.4 [M+H]⁺.

### Dimethyl (2-(2-(4-((4-(3-bromo-4-methoxybenzamido)benzyl)amino)-3-fluorophenyl)benzo[d]oxazol-5-yl)acetyl)glutamate (34).

L-Glutamic acid dimethyl ester hydrochloride (0.13 g, 0.60 mmol); 50 mL; R_{f} (ethyl acetate/methanol 9:1): 0.87; 70% as a yellow-green solid; 182-184°C; IR v 3265 (NH), 1732 (C=O ester), 1673 (C=O amide), 1647 (C=O amide) cm⁻¹; ¹H NMR (dimethylsulfoxide d*₆*) δ 1.75-1.80 (m, 1H, αCH₂), 1.90-2.00 (m, 1H, αCH₂), 2.27 (t, 2H, βCH₂, J = 8 Hz), 3.55-3.65 (m, 8H, ArCH₂CO and COOCH₃), 3.93 (s, 3H, OCH₃), 4.24-4.32 (m, 1H, CH₃OCOCH), 4.44 (br d, 2H, PhCH₂NH), 6.76 (t, 1H, benzene H, J = 8 Hz), 7.11 (br t, 1H, NHCH₂), 7.25 (t, 2H, benzoxazole C6-H and benzoxazole C4-H, J = 8 Hz), 7.36 (s, 1H, benzene H, J = 8 Hz), 7.56-7.61 (m, 3H, benzoxazole C7-H and benzene H), 7.70-7.80 (m, 4H, benzene H), 8.00 (d, 1H, benzene H, J = 8 Hz), 8.22 (s, 1H, benzene H), 8.39 (br d, 1H, CONHCH), 10.17 (s, 1H, Ar-NHCO); MS: m/z (ESI) 761.7 [M+H]⁺.

### Methyl N⁶-(((9H-fluoren-9-yl)methoxy)carbonyl)-N²-(2-(2-(4-((4-(3-bromo-4-methoxybenzamido)benzyl)amino)-3-fluorophenyl)benzo[d]oxazol-5-yl)acetyl)lysinate (35).

N^{ε}-Fmoc-L-lysine methyl ester hydrochloride (0.42 g, 1 mmol), 50 mL; R_{f} (ethyl acetate/methanol 9:1): 0.87; 80% as a yellow solid; 172-174°C; IR v 3293 (NH), 1724 (C=O ester), 1646 (C=O amide), 1622 (C=O amide), 1599 (C=O amide) cm⁻¹; ¹H NMR (dimethylsulfoxide d*₆*) δ 1.18-1.37 (m, 6H, γ-εCH₂ -Lys), 2.93-2.95 (d, H, CH- Fmoc, J =8 Hz), 3.58-3.64 (m, 5H NHCOCH₂-Ar and COOCH₃), 3.94 (s, 3H, OCH₃), 4.16 (br m, 1H, αCH-Lys), 4.29 (d, 2H, O-CH₂-fluorene, J = 8 Hz), 4.44 (d, 2H, CH₂ benzylamine, J = 8 Hz), 6.06 (br s, 1H, NH-Fmoc), 6.75-6.77 (br t, 1H, benzene H), 7.11 (br t, 1H NH benzylamine), 7.21-7.74 (m, 18 H, fluorene H, benzoxazole C6-H, benzoxazole C4-H and benzene H), 7.92 (d, 1H, benzoxazole C7-H, J = 8 Hz), 8.21 (d, 2H, benzene H J = 4 Hz), 8.52 (br s, 1H, Ar-NHCO-Ar); MS: m/z (ESI) 968.5 [M+H]⁺.

### Methyl (2-(2-(4-((4-(3-bromo-4-methoxybenzamido)benzyl)amino)-3-fluorophenyl)benzo[d]oxazol-5-yl)acetyl)lysinate (36).

A round-bottom flask was charged with compound (35) (100 mg, 0.10 mmol) and a solution of 20% (v/v) piperidine in anhydrous N,N-dimethylformamide (freshly prepared) was added in one portion, and the mixture was stirred at room temperature for 30 min under argon atmosphere. Upon reaction completed, the mixture was poured into water and the solid that formed was filtered, washed with hexane and dried under IR lamp to afford the pure product as a white solid. R_{f}(ethyl acetate/chloroform 7:3): 0.19; 100%; 197 °C; IR *v* 3450 (NH₂), 2910 (NH), 1731 (C=O ester), 1623 (C=O amide), 1618 (C=O amide) cm⁻¹; ¹H NMR (dimethylsulfoxide d*₆*) δ 1.35-1.64 (m, 8H, γ-εCH₂ -Lys), 3.62 (s, 2H, NHCOCH₂-Ar), 3.59-3.65 (m, 5H NHCOCH₂-Ar and COOCH₃), 3.93 (s, 3H, OCH₃), 6,75-8.21 (m, 14H, benzoxazole C6-H, benzoxazole C4-H and benzene H and NH benzylamine), 8.52 (br s, 1H, Ar-NHCO-Ar), 10.17 (br s, 1H, Ar-NHCO-Ar); MS: m/z (ESI) 746.6 [M+H]⁺.

### General procedure K (GP-K) to to obtain acid derivatives (37), (38), (39), (40) and (41).

A solution of lithium hydroxide (2.5 mmol) in distilled water to a solution of the appropriate ester (1 mmol) in tetrahydrofuran (fivefold the water amount) was added and the reaction was vigorously stirred overnight. The organic phase was removed under vacuum and the resulting suspension was acidified with 1N HCI until pH 4-5 was obtained. The solid which formed was collected by filtration, then washed with water and dried under IR lamp to afford pure acids. For each compound amount of ester; tetrahydrofuran; R_{f}; yield (%); melting point (°C); IR; ¹H NMR, purity (%) and MS (ESI) are reported.

### (2-(2-(4-((4-(3-bromo-4-methoxybenzamido)benzyl)amino)-3-fluorophenyl)benzo[d]oxazol-5-yl)acetyl)glycine (37) (SST0865AA1).

Compound (31) (0.15 g, 0.22 mmol), 20 mL; R_{f} (ethyl acetate:methanol 7:3): 0.23; 70% as a yellow solid; washed with tetrahydrofuran; IR: *v* 3424 (OH), 3054 (NH), 1726 (C=O acid), 1650, 1597 (C=O amide) cm⁻¹; ¹H NMR (dimethylsulfoxide d*₆*) δ 3.60 (s, 2H, ArCH₂CO), 3.84 (d, 2H, NHCH₂CO, J = 4 Hz), 3.93 (s, 3H, OCH₃), 4.44 (br d, 2H, NHCH₂Ph), 6.76 (t, 1H, benzene H, J = 8 Hz), 7.11 (br t, 1H, NHCH₂), 7.24 (t, 2H, benzoxazole C6-H and C4-H, J = 8 Hz), 7.36 (d, 2H, benzene H, J = 8 Hz), 7.59 (s, 1H, benzene H), 7.62 (d, 1H, benzoxazole C7-H, J = 8 Hz), 7.70-7.80 (m, 4H, benzene H), 8.00 (d, 1H, benzene H, J = 8 Hz), 8.22 (s, 1H, benzene H), 8.51 (t, 1H, NHCH₂CO, J = 4 Hz), 10.18 (s, 1H, NHCO); MS: m/z (ESI) 659.3 [M-H]⁻.

### ((2S)-2-(2-(4-((4-(3-bromo-4-methoxybenzamido)benzyl)amino)-3-fluorophenyl)benzo[d]oxazol-5-yl)acetyl)-alanine (38). (SST0874AA1).

Compound (32) (0.15 g, 0.21 mmol); 20 mL; R_{f}(ethyl acetate/methanol 7:3): 0.18; 70% as a yellow-orange solid; 200°C; washed with tetrahydrofuran; IR *v* 3438 (OH), 3304 (NH), 1732 (C=O acid), 1630 (C=O amide), 1599 (C=O amide) cm⁻¹; ¹H NMR (dimethylsulfoxide d*₆*) δ 1.29 (br d, 3H, CH₃CH), 3.57 (s, 2H, ArCH₂CO), 3.93 (s, 3H, OCH₃), 4.23 (m, 1H, CH₃CH), 4.44 (br d, 2H, NHCH₂Ph), 6.76 (t, 1H, benzene H, J = 8 Hz), 7.11 (br t, 1H, NHCH₂), 7.24 (t, 2H, benzoxazole C6-H and C4-H, J = 8 Hz), 7.36 (d, 2H, benzene H, J = 8 Hz), 7.57 (s, 1H, benzene H), 7.61 (d, 1H, benzoxazole C7-H, J = 8 Hz), 7.70-7.80 (m, 4H, benzene H), 8.00 (d, 1H, benzene H, J = 8 Hz), 8.22 (s, 1H, benzene H), 8.51 (br d, 1H, NHCHCH₃), 10.18 (s, 1H, NHCO), 12.49 (s, 1H, COOH); MS: m/z (ESI) 673.5 [M-H]⁻.

### ((2S)-2-(2-(4-((4-(3-bromo-4-methoxybenzamido)benzyl)amino)-3-fluorophenyl)benzo[d]oxazol-5-yl)acetyl)-phenylalanine (39). (SST0875AA1).

Compound (33) (0.15 g, 0.19 mmol); 20 mL; R_{f}(ethyl acetate/methanol 7:3): 0.17; 60% as an orange solid; 152°C; washed with tetrahydrofuran; IR v 3285 (OH), 2924 (NH) 1723 (C=O acid), 1649 (C=O amide), 1623 (C=O amide) cm⁻¹; ¹H NMR (dimethylsulfoxide d*₆*) δ 2.99 (br d, 2H, CH₂CH), 3.53 (s, 2H, ArCH₂CO), 3.60 (s, 3H, COOCH₃), 4.44-4.50 (m, 1H, CH₂CH), 6.76 (t, 1H, benzene H, J = 8 Hz), 7.05-30 (m, 9H, NHCH₂ and PhCH₂), 7.36 (d, 1H, benzoxazole C6-H, J = 8 Hz), 7.49 (s, 1H, benzoxazole C4-H), 7.56 (s, 1H, benzoxazole C7-H, J = 8 Hz), 7.70-7.80 (m, 4H, benzene H), 8.00 (d, 1H, benzene H, J = 8 Hz), 8.22 (s, 1H, benzene H), 8.41 (br d, 1H, COCHNH), 10.18 (s, 1H, NHCO), 12.60 (s, 1H, COOH); MS: m/z (ESI) 751.3 [M+H]⁺.

### ((2S)-2-(2-(4-((4-(3-bromo-4-methoxybenzamido)benzyl)amino)-3-fluorophenyl)benzo[d]oxazol-5-yl)acetyl)-glutamic acid (40) (SST0876AA1).

Compound (34) (0.15 g, 0.19 mmol); 20 mL; R_{f}(ethyl acetate/methanol 7:3): 0.63; 72% as an orange solid; 197-199°C; washed with tetrahydrofuran; IR *v* 3313 (OH), 2944 (NH) 1716 (C=O acid), 1621 (C=O amide), 1598 (C=O amide) cm⁻¹; ¹H NMR (dimethylsulfoxide d*₆*) δ 1.75-1.80 (m, 1H, αCH₂), 1.90-2.00 (m, 1H, αCH₂), 2.27 (t, 2H, βCH₂, J = 8 Hz), 3.55-3.65 (s, 2H, ArCH₂CO), 3.93 (s, 3H, OCH₃), 4.24-4.32 (m, 1H, CH₃OCOCH), 4.44 (br d, 2H, PhCH₂NH), 6.76 (t, 1H, benzene H, J = 8 Hz), 7.11 (br t, 1H, NHCH₂), 7.25 (t, 2H, benzoxazole C6-H and benzoxazole C4-H, J = 8 Hz), 7.36 (s, 1H, benzene H, J = 8 Hz), 7.56-7.61 (m, 3H, benzoxazole C7-H and benzene H), 7.70-7.80 (m, 4H, benzene H), 8.00 (d, 1H, benzene H, J = 8 Hz), 8.22 (s, 1H, benzene H), 8.39 (br d, 1H, COCHNH), 10.17 (s, 1H, NHCO), 12.50 (s, 2H, COOH); MS: m/z (ESI) 733.4 [M+H]⁺.

### ((2S)-2-(2-(4-((4-(3-bromo-4-methoxybenzamido)benzyl)amino)-3-fluorophenyl)benzo[d]oxazol-5-yl)acetyl)-lysine (41) (SST0916AA1).

Compound (36) (20 mg, 0.027 mmol); 5 mL; R_{f} (ethyl acetate/methanol 1:1): 0.3; 51% as a grey solid; 214-216 °C; washed with tetrahydrofuran; IR v 3439 (various NH), 2940 (OH), 1728 (C=O), 1625 (C=O) cm-1; ¹H NMR (dimethylsulfoxide d*₆*) δ 1.15-1.72 (m, 6H, γ-εCH₂₋Lys), 3.58 (s, 2H, NHCOCH₂-Ar), 3.58 (s, 2H, βCH₂-Lys), 3.93 (s, 3H, OCH₃), 4.16 (br m, 1H, aCH-Lys), 4.43 (br d, 2H, CH₂ benzylamine), 6,76-8.21 (m, 14H, benzoxazole C6-H, benzoxazole C4-H and benzene H and NH benzylamine), 8.37 (br d, 1H, Ar-NHCO-Ar), 10.17 (s, 1H, Ar-NHCO-Ar), 12.5 (br s, 1H, OH); MS: m/z (ESI) 732.0 [M+H]⁺.

### Example 6

### Compound 43

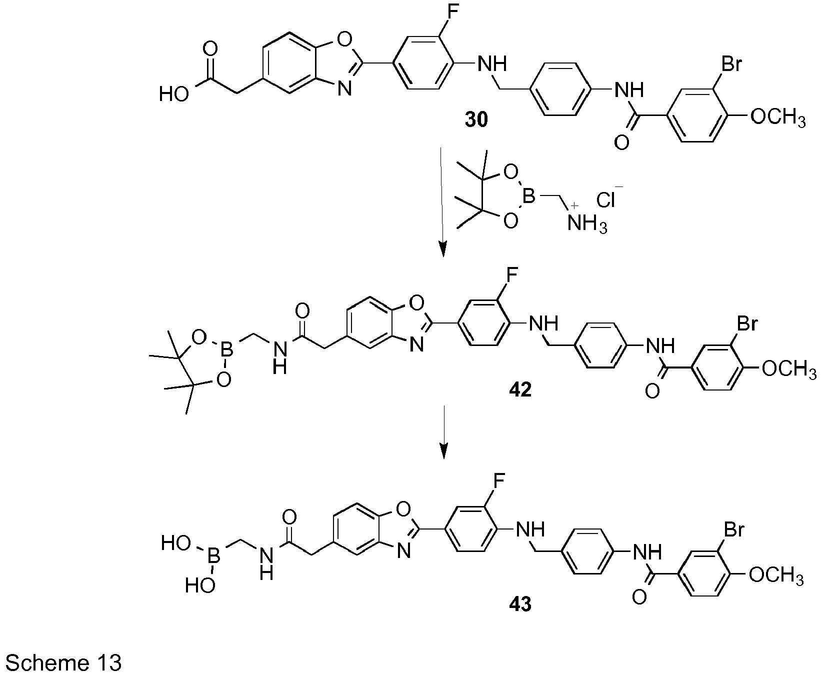

### 3-Bromo-N-(4-(((2-fluoro-4-(5-(2-oxo-2-(((4,4,5,5-tetramethyl-1,3,2-dioxaborolan-2-yl)methyl)amino)ethyl)benzo[d]oxazol-2-yl)phenyl)amino)methyl)phenyl)-4-methoxybenzamide (42).

A mixture of (4,4,5,5-tetramethyl-1,3,2-dioxaborolan-2-yl)methanaminium chloride (0.15 g, 0.80 mmol), compound (30) (0.4 g, 0.66 mmol), and O-(benzotriazol-1-yl)-N,N,N',N'-tetramethyluronium tetrafluoroborate (TBTU) (0.29 g, 0.91 mmol) in anhydrous tetrahydrofuran (30 mL) was cooled to -80° C while stirring. Then a solution of N,N-diisopropylethylamine (DIPEA) (0.31 g, 2.4 mmol, 0.41 mL) in the same solvent (4 mL) was added dropwise in 2 h, while keeping the temperature at -80°C. The mixture was further stirred for 1.5 h, and then slowly heated to room temperature. The solvent was removed under vacuum and the mixture was treated with distilled water, the solid which formed was filtered, washed with water and dried under IR lamp to afford the pure product (0.37 g, 75%) as an orange solid. R_{f} (n-hexane/ethyl acetate 1:1): 0.5; 150-151°C; washed with diethyl ether; IR v 3298 (NH), 1651 (C=O amide), 1621 (C=O amide)cm⁻¹. ¹H NMR (dimethylsulfoxide d*₆*) δ 1.07 (s, 12H, CH₃C), 2.22 (br d, 1H, CH₂B), 3.52 (br d, 1H, CH₂B), 3.70 (s, 1H, ArCH₂CO), 3.94 (s, 3H, OCH₃), 4.44 (br d, 2H, PhCH₂NH), 6.76 (t, 1H, benzene H, J = 8 Hz), 7.11 (br t, 1H, NHCH₂), 7.24 (t, 2H, benzoxazole C6-H and C4-H, J = 8 Hz), 7.36 (d, 2H, benzene H, J = 8 Hz), 7.59 (s, 1H, benzene H), 7.62 (d, 1H, benzoxazole C7-H,, J = 8 Hz), 7.70-7.80 (m, 4H, benzene H), 8.00 (d, 1H, benzene H, J = 8 Hz), 8.22 (s, 1H, benzene H), 9.10 (br t, 1H, CH₂NHCO), 10.18 (s, 1H, NHCO); MS: m/z (ESI) 743.1 [M+H]⁺.

### ((2-(2-(4-((4-(3-Bromo-4-methoxybenzamido)benzyl)amino)-3-fluorophenyl)benzo[d]oxazol-5-yl)acetamido)methyl)boronic acid (43). (SST0877AA1).

A solution of aqueous ammonium acetate (4.4 mL, 0.1 N) and sodium periodate (0.13 g, 0.60 mmol) were added into a well stirred solution of compound (42) (0.15 g, 0.20 mmol) in acetone (5.2 mL). The mixture was vigorously stirred at room temperature overnight. The organic solvent was removed under vacuum and the resulting solid was collected by filtration, washed with water, dried under IR lamp then washed with diethyl ether to afford pure compound (43) as a yellow solid (0.5 g, 38%). R_{f} (ethyl acetate): 0.4; 191-193°C; washed with diethyl ether; IR v 3429 (OH), 3297 (NH), 1651 (C=O amide), 1621 (C=O amide)cm⁻¹. ¹H NMR (dimethylsulfoxide d*₆*) δ 2.20 (br d, 1H, CH₂B), 3.57 (s, 2H, ArCH₂CO), 3.74 (br d, 1H, CH₂B), 3.98 (s, 3H, OCH₃), 4.49 (br d, 2H, ArCH₂NH), 6.81 (t, 1H, benzene H, J = 8 Hz), 7.15 (br t, 1H, NHCH₂), 7.28-7.31 (m, 2H, benzoxazole C6-H and C4-H), 7.40 (d, 2H, benzene H, J = 8 Hz), 7.57-7.62 (m, 2H, benzene H and benzoxazole C7-H), 7.76-7.83 (m, 3H, benzene H), 8.05 (d, 1H, benzene H, J = 8 Hz), 8.27 (s, 1H, benzene H), 8.72 (br t, 1H, CH₂NHCO), 8.83 (br s, 2H, OH), 10.22 (s, 1H, NHCO); MS: m/z (ESI) 661.4 [M+H]⁺.

### Biological assays

### Example 7

### Heparanase inhibition activity

### In vitro screening for heparanase activity

All compounds were tested in a simple, accurate and robust microplate assay based on the cleavage of the synthetic heparin fragment, the pentasaccharide Fondaparinux (AGA*IA). This assay was originally developed by Hammond and coworkers [Hammond E, Li CP, Ferro V. Development of a colorimetric assay for heparanase activity suitable for kinetic analysis and inhibitor screening. Anal. Biochem. 396(1), 112-116 (2010)], where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 427(1), 82-89 (2012)].

Compounds 11 (SST0914AA1) and 29 (SST0915AA1), whose synthesis are reported above, were used as reference compounds.

### Materials and methods

To determine the activity of the newly designed heparanase inhibitors an homogenous assay based on the cleavage of the synthetic heparin oligosaccharide fondaparinux has been employed (Hammond E. et al. Anal. Biochem. 2010, 396, 112). The assay measures the appearance of the disaccharide product of heparanase-catalyzed fondaparinux cleavage colorimetrically using the tetrazolium salt WST-1. Assay was essentially performed as described with minor modifications. Briefly, NUNC ELISA 96-well plates were pre-treated with a solution of 4% BSA (bovin serum albumin) in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

IC₅₀ value for each compound, versus heparanase, was calculated by GraphPad software and results are summarized in Table 2, below. Finally, the measurements were corrected by subtracting both the reagent background and the inner absorbance value of test compound.

**Table 2. IC₅₀ value in AGA*IA assay, versus heparanase inhibition of selected compounds of present invention.**

| Compound | Code lab | AGA*IA assay (IC₅₀) |
|---|---|---|
| Reference compound | Roneparstat (SST0001) | ∼ 5 nM |
| Reference compound | Suramin | ∼ 26 µM |
| 22 | SST0623AA1 | +++ |
| 30 | SST0864AA1 | + |
| 37 | SST0865AA1 | + |
| 10 | SST0867 AA 1 | + |
| 16 | SST0868AA1 | + |
| 17 | SST0869AA1 | + |
| 15 | SST0871AA1 | ++ |
| 19 | SST0873AA1 | + |
| 39 | SST0875AA1 | + |
| 40 | SST0876AA1 | ++ |

| | | |
|---|---|---|
| Legend: +: 1.00-20.00 µM ++: 0.50-0.99 µM +++: 0.10-0.49 µM | | |

From the results with a first screening on AGA*IA it is quite evident that the compounds of the present invention are endowed with high anti-heparanase activity. Some of them are active in the micromolar range (more potent than suramin), others are active in a submicromolar range. SST0623AA1 showed an anti-heparanase activity at very low concentration, less than 0.5 µM.

Given the difference of M.W. with heparin-derivatives macromolecules currently under clinical investigation, it is very advantageous that these compounds are potent as Roneparstat, herein used as reference compound. Roneparstat, used as reference compound, is a potent inhibitor of heparanase currently undergoing clinical trials.

Also, the effect on the heparanase activity of fluorine and non-fluorine derivatives was studied.

Results are shown in Table 3 below.

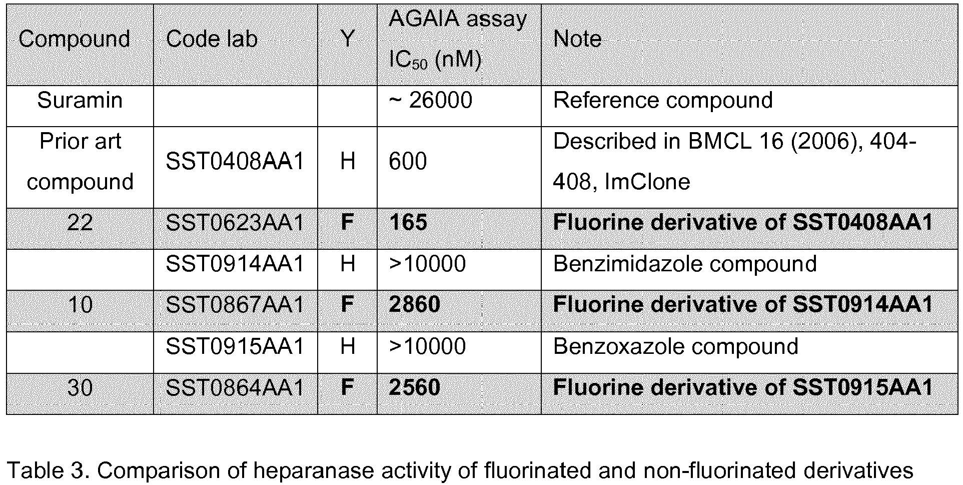

It is evident from the above table 3 that the introduction of a fluorine instead of an hydrogen in position 3 of phenyl ring linked to azole moiety, increases the heparanase inhibitory activity of the compound with respect to the corresponding unsubstituted compound (Y=H). Compounds SST0914AA1 and SST0915AA1 are not within the scope of the present invention but they are used as reference compounds to show the positive effect of the introduction of fluorine on heparanase inhibition activity.

Upon screening as inhibition of heparanase enzymatic activity, selected active compounds were furtherly characterized *in vitro,* through a suitable cell proliferation assay (SRB Cell Cytotoxicity Assay) for their putative cytotoxic activity against three tumor cell lines, and through specific cellular assays for their putative anti-metastatic activity. According to these data selected compounds were also tested for the effects on real-time quantitative PCR.

### Example 8

### Cytotoxicity assay

### Materials and methods

SRB Cell Cytotoxicity Assay is an assay based upon the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

### Results

The following Table 4 reports the percentage of tumor growth inhibition (% TGI) versus control of the tested compounds.

Known compound SST0408AA1 (BMCL 16 (2006), 404-408, ImClone) was used as reference compound.

**Table 4. Putative anti-proliferative activity of the selected compounds on three tumor cell lines. N.I.: Not Inhibition**

| Compound | Code lab | | Concentration of test compounds at which the cell growth is inhibited by 50 % | | |
|---|---|---|---|---|---|
| | | Conc. (µM) | HT1080 | U87MG | U2OS |
| Reference | SST0408AA1 | 10.0 | 6.0 | 23.0 | 8.7 |
| 22 | SST0623AA1 | 10.0 | 14.2 | 13.1 | 2.7 |
| " | " | 2.5 | N. I. | N. I. | N. I. |
| 37 | SST0865AA1 | 2.5 | N. I. | N. I. | N. I. |
| 15 | SST0871AA1 | 2.5 | N. I. | N. I. | N. I. |
| 40 | SST0876AA1 | 2.5 | N. I. | N. I. | N. I. |

The reference product (SST0408AA1), analogue non-fluorinated of SST0623AA1, is slightly more potent than SST0623AA1 in inhibiting cells growth. Indeed, at the same concentration (10 µM), the fluorinated analogue SST0623AA1 interferes less with the proliferation. Tested at lower concentration (2.5 µM) SST623AA1 as well as the other tested compounds do not interfere with the cell growth on all tumor cell lines tested.

These data, combined with the data reported in the above Table 2, show that compounds of the present invention, at concentrations in which they do not have any interference with cell proliferation, are selective heparanase inhibitors. This makes their use particularly advantageous in conditions wherein an anti-heparanase activity is desired but a cytotoxic effect is undesirable.

### Example 9

### Invasion assay

### Materials and methods

Heparanase inhibitors were then evaluated for their activity on the invasive potential of HT1080 (human fibrosarcoma), U87MG (human glioblastoma astrocytoma) and U2OS (human osteosarcoma) human cell lines, using the Matrigel cell invasion assay which allows to assess the cell invasion ability of malignant and normal cells.

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01M Tris pH 8.0, 0.7% NaCl) (Becton-Dickinson Sciences). DMEM (Dulbecco's Modified Eagle's Medium) containing 5% FBS was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration for several fields, in duplicates. Activity of test compounds was compared to the reference compound. Activity of most interesting compound was confirmed in a second cell invasion assay. Data were expressed as the percent invasion with respect to cell migration through uncoated insert membrane.

### Results

Results are summarized in the following Table 5.

**Table 5. Inhibition of invasive potential of HT1080 (human fibrosarcoma), U87MG (human glioblastoma astrocytoma) and U2OS (human osteosarcoma) human cell lines of selected active compounds.**

| Cpd | Code lab | Conc. | Invasion Assay | | |
|---|---|---|---|---|---|
| | | | HT1080 | U87MG | U2OS |
| **22** | **SST0623AA1** | 1.1µM | ++ | ++ | ++ |
| **15** | **SST0871AA1** | 2.5µM | + | + | + |
| **40** | **SST0876AA1** | 2.5µM | ++ | + | ++ |
| Ref. | Suramin | 10µM | + | - | - |

| | | | | | |
|---|---|---|---|---|---|
| Legend ++: 50-90% + : < 50% - : Not Active | | | | | |

Using an invasion assay, we have demonstrated that selected compounds, evaluated at not cytotoxic concentrations, were able to inhibit invasion of three tumor cell lines, more than suramin.

### Example 10

### Real-Time Quantitative PCR (qPCR) for measuring mRNA gene-expression of FGF1/2, VEGF, MMP-9, HSPE-1 in genes tumor cell lines

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR.

### Materials and methods

To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate. In Table 6 a mean value is reported.

### Results

Results are summarized in the following Table 6.

**Table 6. Real-Time RT-PCR of pro-angiogenic genes**

| | | Gene Expression (% mRNA vs. control) | | | | |
|---|---|---|---|---|---|---|
| | Conc | FGF-1 | FGF-2 | VEGF | MMP-9 | HPSE-1 |
| Control | | ∼ 100 | ∼ 100 | ∼ 100 | ∼ 100 | ∼ 100 |
| SST0408AA1 | 1.0 uM | ∼ 97 | ∼ 92 | ∼ 72 | ∼ 69 | ∼ 98 |
| SST0623AA1 | 1.0 uM | ∼ 77 | ∼ 71 | ∼ 67 | ∼ 52 | ∼ 82 |
| SST0899AA1 | 2.5 uM | ∼ 53 | ∼ 82 | ∼ 55 | ∼ 50 | ∼ 63 |
| Reference compound (SST0001) | 10 uM | ∼ 70 | ∼ 77 | ∼ 43 | ∼ 42 | ∼ 72 |

Data from qPCR assay, with the two selected active compounds SST0623AA1, analogue fluorinated of SST0408AA1, and SST0899AA1, versus SST0001 (reference compound) on HT1080 (human fibrosarcoma) cells highlighted an inhibitory effect against the tumor transcription genes encoding for all proangiogenic factors tested.

From the comparison of the results of SST0623AA1 versus SST0408AA1, the analogue fluorinated object of present invention is more active that corresponding non fluorinated compound. Compound SST0899AA1, tested at 2.5 µM, is also active in inhibition of tumor transcription genes encoding for all proangiogenic factors tested (FGF1/2, VEGF, MMP-9, HSPE-1).

The reference compound SST0001, tested at 10 µM, is also active.

## Claims

1. A compound having the following formula (I) 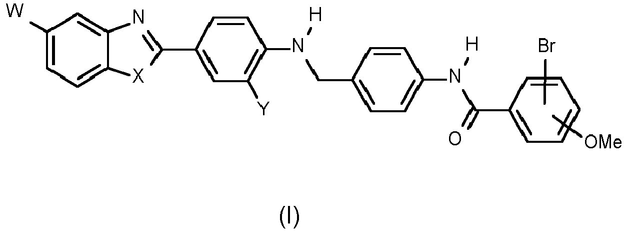 wherein
Y is fluorine,
X is selected from the group consisting of O and NH,
W is selected from the group consisting of H and CH₂COZ, wherein Z is selected from the group consisting of OH, NH-CHR-COOH, wherein R is the residue of an amino acid, and NHCH₂B(OH)₂,
the enantiomers, diastereoisomers, and mixtures thereof,
or a pharmaceutically acceptable salt, hydrate or solvate thereof.

2. The compound according to claim 1 wherein W is CH₂COZ and Z is NH-CHR-COOH, wherein R is a residue of an amino acid selected from glycine, alanine, phenylalanine and glutamic acid.

3. The compound according to claim 1 wherein X is NH.

4. The compound according to claim 3 wherein W is H.

5. The compound according to claim 3 wherein W is CH₂COZ wherein Z is NH-CHR-COOH and R is an amino acid residue.

6. The compound according to claim 5 wherein R is a residue of glycine.

7. The compound according to claim 1 wherein X is O.

8. The compound according to claim 7 wherein W is CH₂COZ wherein Z is NH-CHR-COOH and R is an amino acid residue.

9. The compound according to claim 8 wherein R is a residue of glutamic acid.

10. The compound according to anyone of the preceding claims which is one of the followings:
2-(4-(4-(3-bromo-4-methoxybenzamido)benzyl)amino)-3-fluorophenyl)-1*H-*benzo[d]imidazole,
2-(2-(4-((4-(3-bromo-4-methoxybenzamido)benzyl)amino)-3-fluorophenyl)benzo[d]oxazol-5-yl)acetic acid,
(2-(2-(4-((4-(3-bromo-4-methoxybenzamido)benzyl)amino)-3-fluorophenyl)benzo[d]oxazol-5-yl)acetyl)glycine,
2-(2-(4-((4-(3-bromo-4-methoxybenzamido)benzyl)amino)-3-fluorophenyl)-1*H-*benzo[d]imidazol-5-yl)acetic acid,
(2S)-2-(2-(2-(4-((4-(3-bromo-4-methoxybenzamido)benzyl)amino)-3-fluorophenyl)-1*H-*benzo[d]imidazol-5-yl)acetamido)propanoic acid,
(2S)-2-(2-(2-(4-((4-(3-bromo-4-methoxybenzamido)benzyl)amino)-3-fluorophenyl)-1*H-*benzo[d]imidazol-5-yl)acetamido)-3-phenylpropanoic acid,
((2-(2-(4-((4-(3-Bromo-4-methoxybenzamido)benzyl)amino)-3-fluorophenyl)-1*H-*benzo[d]imidazol-5-yl)acetamido)methyl)boronic acid,
2-(2-(2-(4-((4-(3-bromo-4-methoxybenzamido)benzyl)amino)-3-fluorophenyl)-1*H-*benzo[d]imidazol-5-yl)acetamido)acetic acid and
((2S)-2-(2-(4-((4-(3-bromo-4-methoxybenzamido)benzyl)amino)-3-fluorophenyl)benzo[d]oxazol-5-yl)acetyl)-glutamic acid.

11. The compound according to anyone of claims 1-9 which is one of the followings:
((2S)-2-(2-(4-((4-(3-bromo-4-methoxybenzamido)benzyl)amino)-3-fluorophenyl)benzo[d]oxazol-5-yl)acetyl)-alanine,
((2S)-2-(2-(4-((4-(3-bromo-4-methoxybenzamido)benzyl)amino)-3-fluorophenyl)benzo[d]oxazol-5-yl)acetyl)-phenylalanine,
((2S)-2-(2-(4-((4-(3-bromo-4-methoxybenzamido)benzyl)amino)-3-fluorophenyl)benzo[d]oxazol-5-yl)acetyl)-lysine, and
((2-(2-(4-((4-(3-Bromo-4-methoxybenzamido)benzyl)amino)-3-fluorophenyl)benzo[d]oxazol-5-yl)acetamido)methyl)boronic acid.

12. The compound of anyone of claims 1-11 for use as a medicament.

13. The compound of anyone of claims 1-11 for use in the treatment of a disease which can be improved or prevented by an anti-heparanase activity.

14. The compound for the use according to claim 13 wherein said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

15. A pharmaceutical composition containing as active ingredient a compound of anyone of claims 1-11 and at least one pharmaceutically acceptable vehicle and/or excipient.

16. The pharmaceutical composition according to claim 15 wherein said composition further comprises one or more further active ingredients.

17. The pharmaceutical composition according to claim 16 wherein said further active ingredient is a chemotherapeutic agent.

18. The pharmaceutical composition according to claim 17 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

19. The pharmaceutical composition according to claim 18 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
